# EUROPEAN PATENT APPLICATION

(11) **EP 2 966 170 A1**
(43) Date of publication of application: **13.01.2016**
(21) Application number: 14176629.5
(22) Date of filing: 10.07.2014
(51) Int. Cl.: C12N 15/10, C12N 15/113, A61K 31/7088, A61P 31/20

(54) **HBV inactivation**

(71) Applicant: Heinrich-Pette-Institut Leibniz-Institut für experimentelle Virologie-Stiftung bürgerlichen Rechts -, 20251 Hamburg (DE); Universitätsklinikum Hamburg-Eppendorf, 20246 Hamburg (DE); Technische Universität Dresden, 01062 Dresden (DE)
(72) Inventor: Hauber, Joachim, 22527 Hamburg (DE); Karimova, Madina, 01099 Dresden (DE); Schulze zur Wiesch, Julian, 22589 Hamburg (DE); Buchholz, Frank, 01307 Dresden (DE)
(74) Representative: UEXKÜLL & STOLBERG

(57) **Abstract**

The present invention relates to a DNA-binding molecule which is able to specifically bind to a predetermined nucleotide sequence within the hepatitis B virus genome. The DNA-binding molecule is suitable for use in a method of treating and/or preventing a hepatitis B and/or a hepatitis D virus infection in a patient. The invention also relates to a method for treating and/or preventing a hepatitis B and/or a hepatitis D virus infection in a patient which makes use of the DNA-binding molecule. The invention also relates to vectors, kits and compositions comprising the DNA-binding molecule.

## Description

### SUMMARY OF THE INVENTION

The present invention relates to a DNA-binding molecule which is able to specifically bind to a predetermined nucleotide sequence within the hepatitis B virus genome. The DNA-binding molecule is suitable for use in a method of treating and/or preventing a hepatitis B and/or a hepatitis D virus infection in a patient. The invention also relates to a method for treating and/or preventing a hepatitis B and/or a hepatitis D virus infection in a patient which makes use of the DNA-binding molecule. The invention also relates to vectors, kits and compositions comprising the DNA-binding molecule.

### TECHNICAL BACKGROUND

Hepatitis B virus (HBV) is a small, enveloped DNA virus belonging to the family of the *Hepadnaviridae* that infects millions of patients worldwide. According to the WHO, an estimated 2 billion people have undergone HBV infection so that about 5% of the world population (350 million people) are presently chronically infected (Medley et al., 2001, Nat Med, 7:619-24). More than 500,000 people die every year related to the HBV infection (Ganem and Prince, 2004, N Engl J Med, 350: 1118-29; Medley et al., 2001, Nat Med, 7:619-24). Pronounced differences in regional distribution of HBV infections can be observed. For example, while less than 0.5% of the population in Northern Europe are chronically infected with the hepatitis B virus, up to 8% of the population in Turkey are affected.

Most cases of acute hepatitis B infection in adults (>90%) resolve leading to a life-long immune control, although the virus can theoretically still reactivate in these patients, e.g. during chemotherapy. In up to 10% of acutely HBV-infected adults, about 90% of young children and 30-90% of immune-compromised persons, the infection evolves into a chronic form (Ganem and Prince, 2004, N Engl J Med, 350: 1118-29; Medley et al., 2001, Nat Med, 7:619-24). The considerable public health significance of hepatitis B primarily results from the consequences of these chronic infections, in particular the development of cirrhosis of the liver and/or hepatocellular carcinoma (HCC). Chronic HBV infection is asymptomatic in many patients for long periods of time but can lead to active chronic liver disease at any point and subsequently lead to cirrhosis and hepatocellular carcinoma (Wong and Chan, 2012, Ann Hepatol, 11:284-93). HBV infection is responsible for most cases of hepatocellular carcinoma worldwide with a rising incidence. In addition, there are a number of extra-hepatic manifestations of chronic HBV infection like glomerulonephritis (Liaw and Chu, 2009, Lancet, 373:582-92). HBV is also a major indication for liver transplantation with the risk of re-infection of the transplanted liver. Many patients with chronic hepatitis B infection are therefore potential candidates for antiviral therapy (Cornberg et al., 2011, Z. Gastroenterol, 49:871-930).

The HBV genome consists of a circular, partially double stranded DNA, which is synthesized through an RNA intermediate, with the aid of a reverse transcriptase. The virus particle consists of an outer lipid envelope consisting of the hepatitis B surface antigen (HBsAg). This envelope encloses an icosahedral nucleocapsid core composed of the core antigen (HBcAg) (Ganem and Prince, 2004, N Engl J Med, 350: 1118-29). The nucleocapsid encloses the viral DNA and a DNA polymerase that has reverse transcriptase activity. HBV establishes its genome in human liver cells as episomal covalently closed circular (ccc) DNA. Thus, a viral reservoir for HBV reactivation is created that makes the lasting HBV therapy extremely difficult (Dienstag, 2008, N Engl J Med, 359:1486-500; Liaw and Chu, 2009, Lancet, 373:582-92).

Moreover, simultaneous or super-infection with hepatitis D virus (HDV) can occur in HBV infected patients. The HDV can only assemble as an infectious virus particle when it is supplemented with the HBsAg envelope of HBV. Eradication of the HBV cccDNA reservoir would therefore also represent a new concept for the treatment of HDV infection. A HDV superinfection of a HBV carrier leads to an enhanced liver disease as compared to HBV mono-infection. HDV superinfection becomes chronic in about 90% of cases. It leads to an increased incidence of cirrhosis and an earlier occurrence of hepatocellular carcinoma.

Complete elimination of the hepatitis B virus from the body in chronic hepatitis B infections is not possible by means of the current therapy principles. Vaccination is therefore at present the most important measure to prevent infection and reduce the number of individuals carrying the virus. Nevertheless, in a small minority of patients HBV therapy can induce a seroconversion and subsequent immunological control of the infection. Notably, the secondary aims of HBV treatment are prevention of spreading of the infection, stopping viral replication and liver inflammation and thereby at least reducing the risk of developing cirrhosis or hepatocellular carcinoma.

Current therapy principles include the expensive and laborious therapy with pegylated interferon-α and a number of nucleoside and nucleotide analogues (Dienstag, 2008, N Engl J Med, 359:1486-500; Bhattacharya and Thio, 2010, Clin Infect Dis, 51:1201-8). Interferon-α or pegylated interferon-α may only be used in therapy for a limited time (Cornberg et al., 2011, Z. Gastroenterol, 49:871-930), and frequently leads to serious side-effects. Also in cases of a HDV superinfection, the only established therapy is the pegylated interferon-α therapy, which does not have good long-term success rates. Overall, only a small percentage of patients show a long lasting response (Hughes et al., 2011; Wedemeyer et al., 2011). In cases in which interferon therapy is not possible or the patient did not respond, expensive long-term nucleos(t)ide analogue therapy is used for HBV therapy (eg. with Lamivudin, Entecavir or Tenofovir) (Cornberg et al., 2011, Z. Gastroenterol, 49:871-930). Nucleos(t)ide analogue therapy suppresses replication of the HBV virus by inhibition of the viral polymerase. The treatment reduces the probability for development of HCC, but does not eliminate the risk completely. Nowadays, substances with a high genetic barrier to resistance are preferred (eg, Entecavir, Tenofovir), but next to the risk of long-term drug toxicities there is still a limited risk of developing viral drug escape mutations, especially in patients with poor compliance.

Moreover, these drugs are still quite expensive and must be taken almost permanently (for years or even decades) (Bhattacharya and Thio, 2010, Clin Infect Dis, 51:1201-8). In most cases HBV viremia and the liver transaminases rise again after cessation of nucleos(t)ide analogue treatment, sometimes leading to the clinical picture of a HBV reactivation. Regular monitoring of the HBV DNA every 3-6 months to verify treatment success and predict drug resistance in time is therefore necessary. The ultimate therapy goal of a lasting seroconversion from HBsAg (hepatitis B surface antigen) to anti-HBs antibodies is only reached by nucleos(t)ide analogues in 5% to a maximum of 10% of the cases. Current treatment recommendations can be found in the German HBV guidelines (Cornberg et al., 2011, Z. Gastroenterol, 49:871-930).

It can be seen from the above that current therapies employing interferon-α and/or nucleoside/nucleotide analogues can only control viremia, but are unsuitable to eliminate the viral genomic DNA. Consequently, there is a need for new therapy approaches which ultimately result in a complete inactivation and eradication of the virus from the infected patient. Such treatment would provide a cure for millions of chronically HBV infected patients worldwide.

### DESCRIPTION OF THE INVENTION

The present invention provides a highly effective and specific method of targeting the HBV cccDNA as well as integrated HBV genome, thereby introducing new possibilities to inactivate the HBV genome both *in vitro* and in HBV-infected patients.

### DNA-binding molecule for use in the treatment and/or prevention of HBV/HDV infection

In a first aspect, the invention relates to a DNA-binding molecule, and in particular an RNA molecule, which is able to specifically bind to a predetermined nucleotide sequence within the hepatitis B virus genome for use in a method of treating and/or preventing hepatitis B and/or a hepatitis D virus infection in a patient, wherein said method comprises the administration of the DNA-binding molecule and a nuclease. The nuclease is guided by the DNA-binding molecule of the present invention to said predetermined nucleotide sequence within the hepatitis B virus genome where it cleaves the DNA to disrupt and/or inactivate the viral genome. The inventors therefore found an easy and reproducible was to inactivate the HBV genome.

The DNA-binding molecule which is able to specifically bind to a predetermined nucleotide sequence within the hepatitis B virus genome can comprise e.g. a) a DNA-binding nucleic acid sequence that is able to specifically bind to the predetermined sequence in the hepatitis B virus genome, or b) a DNA-binding polypeptide sequence that is able to specifically bind to the predetermined sequence in the hepatitis B virus genome. In a preferred embodiment, the DNA binding molecule comprises a DNA-binding nucleic acid sequence that is able to specifically bind to the predetermined sequence in the hepatitis B virus genome.

The person skilled in the art is able to determine whether the binding of two molecules to each other is specific. For example, a "specific" binding in the sense of the present invention means that the DNA-binding molecule and/or the DNA-binding nucleic acid sequence or polypeptide sequence comprised therein has an affinity to bind to the predetermined nucleotide sequence within the HBV genome that is at least 10x, 50x, 100x, 500x, 1000x, or more times higher than the affinity for binding to other nucleotide sequences, preferably non-viral sequences, and more preferably human sequences. Preferably, the DNA-binding molecule and/or the DNA-binding nucleic acid sequence or polypeptide sequence comprised therein has a binding affinity to the predetermined nucleotide sequence within the HBV genome that is at least 100x higher than the binding affinity to other nucleotide sequences.

Where the DNA-binding molecule comprises a DNA-binding nucleic acid sequence, such as an RNA sequence, specific binding will be achieved if said DNA-binding nucleic acid sequence is sufficiently complementary to the predetermined nucleotide sequence in the hepatitis B virus genome, i.e. to the "target sequence". In a preferred aspect, the DNA-binding molecule comprises a DNA-binding nucleic acid sequence, more preferably an RNA sequence, which is at least 70%, 80%, 90% or 95% complementary to the predetermined nucleotide sequence in a sequence stretch of at least 16, 17, 18, 19 or 20 nucleotides, i.e. from 16 to 20 nucleotides. In one embodiment, the DNA-binding molecule comprises a DNA-binding nucleic acid sequence that is complementary to the predetermined nucleotide sequence within the hepatitis B virus genome whereby hybridization of the DNA-binding nucleic acid sequence with the predetermined nucleotide sequence within the hepatitis B virus genome is allowed.

Stated differently, the DNA-binding nucleic acid sequence in the DNA-binding molecule that is able to specifically bind to the predetermined sequence in the hepatitis B virus genome does not need to be fully complementary, but may comprise up to 0, 1, 2, 3, 4 or 5 mismatches within a sequence stretch of at least 16, 17, 18, 19 or 20 nucleotides, i.e. from 16 to 20 nucleotides, preferably less than 3, less than 2 or no mismatches, in comparison to a sequence that is complementary to the predetermined nucleotide sequence in the HBV genome.

The DNA-binding nucleic acid sequence that is able to specifically bind to a predetermined sequence in the hepatitis B virus genome can be DNA or RNA. Preferably, the nucleic acid sequence is RNA. More preferably, the entire DNA-binding molecule comprising the DNA-binding nucleic acid sequence according to the invention is an oligoribonucleotide, i.e. an RNA molecule. The oligoribonucleotide preferably has a size of at least 70 nucleotides, preferably at least 80, 90, 100, 110, 120, 130, 140, 150, 160, 170, 180 or 190 nucleotides. It is preferred that the oligoribonucleotide of the invention comprises less than 200 nucleotides.

In one embodiment, the DNA-binding molecule comprises a CRISPR RNA (crRNA) or a functional variant thereof. CRISPR (clustered regularly interspaced short palindromic repeats) RNAs are well known in the art as part of CRISPR/Cas systems. The original bacterial CRISPR/Cas systems comprise a first processed RNA transcript, that originates from a so-called CRISPR array in the bacterial genome (containing short DNA segments from invading viruses and plasmids), wherein the RNA transcript contains a crRNA comprising a guide RNA (gRNA) sequence as described elsewhere herein and a repeat; a second RNA with partial complementarity to the repeat (tracrRNA, i.e. trans-activating crRNA), and the nuclease Cas9. Complementary base pairing of the repeats in crRNA and tracrRNA leads to formation of a crRNA:tracrRNA duplex. The gRNA comprised by the crRNA is able to bind to the target DNA and the bulk of the tracrRNA binds to Cas9.

Accordingly, it is preferred herein that the DNA-binding molecule of the invention comprises a crRNA sequence, wherein said crRNA sequence comprises a gRNA sequence, the latter of which provides for binding to the predetermined HBV sequence, and a repeat sequence. crRNAs are described in the scientific literature and the person skilled in the art is therefore readily able to design and synthesize suitable crRNAs based on the specific DNA-binding sequences herein. In addition, there are several CRISPR/Cas systems commercially available from different manufacturers which allow for the construction of suitable crRNA molecules. The repeat part in the crRNA will be designed to be sufficiently complementary to a corresponding repeat in a tracrRNA to allow hybridization between the crRNA and the tracrRNA.

Within HBV-infected cells the repeat part of the crRNA sequence will hybridize to the tracrRNA to form a crRNA:tracrRNA duplex. The term "Duplex" in this context is to be understood as RNA double strand. The crRNA:tracrRNA duplex, which is formed from the crRNA and the tracrRNA, can comprise a single RNA molecule or two separate RNA molecules. In one embodiment of the invention, the crRNA and the tracrRNA are separate molecules which means that a crRNA molecule comprising the gRNA and the typical repeat is used in combination with a separate tracrRNA. Hybridization of the two separate RNA molecules results in the crRNA:tracrRNA duplex which enables binding of a nuclease, such as Cas9 or functional fragments or variants thereof.

In a preferred embodiment, the crRNA:tracrRNA duplex comprises a single RNA molecule, i.e. a molecule that comprises both crRNA and the tracrRNA. In a particularly preferred embodiment, the DNA-binding molecule of the invention comprises or consists of a single guide RNA (sgRNA). As used herein, a "single guide RNA" or "sgRNA" is an engineered chimeric RNA molecule in which a crRNA and a tracrRNA are linked by a connecting RNA sequence, such as a sequence forming a loop structure. sgRNA molecules, which is also referred to as synthetic or short guide RNA in the literature are described, e.g. in Jinek et al., 2012, Science, 337:816-21. Preferably, the sgRNA has a sequence selected from the group consisting of SEQ ID NOs:1-4, or a sequence complementary thereto. Consequently, the DNA-binding molecule comprises or consists of a sequence set forth in SEQ ID NO:1-4 or variants or complements thereof. These sgRNAs are encoded by the sequences depicted in SEQ ID NOs:5-8, respectively, or sequences complementary thereto.

Suitable functional variants of DNA-binding molecules that comprise a crRNA in the sense of the present invention are variants that have all the identifying characteristics of the above described DNA-binding molecules that comprise a crRNA. Functional variants of the disclosed DNA-binding molecules that comprise a crRNA, thus, comprise a crRNA sequence comprising a guide RNA (gRNA) sequence as described elsewhere herein, and are, preferably, able to bind to the Cas9 nuclease or variants of the Cas9 nuclease, such as Cas9n. Preferably, these variants are functional variants of a sgRNA sequence selected from the group consisting of SEQ ID NOs:1-4, or a sequence complementary thereto.

Designing sgRNAs from other CRISPR systems than the Type II CRISPR-Cas systems, e.g. from Type I or Type III CRISPR-Cas systems is also possible and such sgRNAs could also be used as DNA-binding molecules (an overview is provided by Terns and Terns, 2014, Trends Genet, 30:3). However, in a preferred embodiment, the sgRNA is derived from a Type II CRISPR-Cas system. The sgRNA is derived from a CRISPR-Cas system that targets DNA.

According to the present invention, the gRNA provides for the specific binding of the DNA-binding molecule of the invention to the predetermined HBV sequence. Preferably, the gRNA has a sequence selected from the group consisting of SEQ ID NOs:9-12, or variants or complements thereof. However, it is also possible that the gRNA has a sequence that is at least 70%, 80%, 90% or 95% identical to a sequence selected from the group consisting of SEQ ID NOs:9-12, or variants or complements thereof. In other words, the gRNA can have a sequence that may comprise up to 0, 1, 2, 3, 4 or 5 mismatches compared with a sequence selected from the group consisting of SEQ ID NOs:9-12, or a sequence complementary thereto, preferably less than 3, less than 2 or no mismatches. More preferably, the gRNA comprises a nucleic acid sequence that is able to specifically bind to a sequence in the hepatitis B virus genome selected from the group consisting of SEQ ID NO:13-16, or a sequence complementary thereto.

In a particularly preferred sgRNA, the gRNA is followed (at its 3' end) by approximately 80 nucleotides of hybrid crRNA/tracrRNA, i.e. an RNA sequence that comprises both the crRNA and the tracrRNA (Carroll, 2014, Annu Rev Biochem, 83:14.1-14.31), such as by SEQ ID NO:17 or functional variants thereof. The hybrid crRNA/tracrRNA binds to the nuclease Cas9. Functional variants of the aforementioned hybrid crRNA/tracrRNA shown in SEQ ID NO:17 are, thus, characterized in that they maintain the ability to bind Cas9 nuclease and/or functional variants of the Cas9 nuclease, such as Cas9n.

GUUUUAGAGC UAGAAAUAGC AAGUUAAAAU AAGGCUAGUC CGUUAUCAAC
UUGAAAAAGU GGCACCGAGU CGGUGCUUUU UU (SEQ ID NO:17).

In this embodiment, the gRNA sequence is the nucleic acid sequence that is able to specifically bind to a sequence in the hepatitis B virus genome defined elsewhere herein. Furthermore, in one embodiment the gRNA sequence is be preceded by a guanine ribonucleotide (G). Such a guanine ribonucleotide may be present to enhance expression of a sgRNA by RNA polymerase III (polIII) from a DNA vector. Thus, the sgRNA of the present invention preferably comprises the following sequence: 
G (N)17-20 GUUUUAGAGC UAGAAAUAGC AAGUUAAAAU AAGGCUAGUC
CGUUAUCAAC UUGAAAAAGU GGCACCGAGU CGGUGCUUUU UU (SEQ ID NO:
18),
 wherein "(N)₁₇₋₂₀" is the gRNA sequence having preferably a length of from 17 to 20 nucleotides.

As used herein "N" or "n" can be any (ribo)nucleotide, i.e. adenine (A), guanine (G), cytosine (C) or thymine (T)/uracil (U), unknown, or other. Preferably, nucleotides designated "N" in SEQ ID NO: 18 are independently selected from the group consisting of a, g, c or u. The crRNA/tracrRNA part of the sgRNA contains in itself a repeat and anti-repeat sequences that form a hairpin RNA structure; further short repeats allow the hybrid to form 3 stemloops (Ni-shimasu, 2014, Cell, 156 (5): 935-049). Thus, in one embodiment, the DNA-binding molecule comprises a crRNA and/or a tracrRNA sequence, preferably a crRNA/tracrRNA hybrid sequence, such as SEQ ID NO:17.

The DNA-binding nucleic acid sequence that is able to specifically bind to a sequence in the hepatitis B virus genome (e.g. the gRNA) has a length of at least 16, 17, 18, 19 or 20 nucleotides (nt), i.e. from 16 to 20 nucleotides. Preferably, said sequence has a length of at least 17 nt. Thus, said sequence can have a length of e.g. 17, 18, 19, or 20 nt. In other words, the sequence preferably has a length from 17 to 20 nt. It is known in the art that nucleotides in excess of 20 in said sequence do not significantly improve the binding. Nevertheless, sequences that are able to specifically bind to a sequence in the hepatitis B virus genome which are longer than 20 nt are allowable in the context of the present invention.

In one embodiment, the DNA-binding nucleic acid sequence is an oligoribonucleotide which comprises less than 200 nucleotides. Preferably, the DNA-binding nucleic acid sequence comprised in the DNA-binding molecule has a length of 16 to 20 nucleotides.

It is furthermore known in the art that extensive, but not complete complementarity between guide RNA sequences and their target sequences is necessary for targeting. The gRNAs are in general tolerant of 1-3 mismatches in their target (Mali et al., 2013, Nat Meth, 10(10):957-963). This mismatch tolerance is an advantage for the disruption of HBV sequences that could otherwise evade the treatment by mutation. Consequently, the guide RNA sequence can comprise 1, 2, 3, 4 or 5 ribonucleotides that are not complementary to the predetermined sequence in the HBV genome. Preferably, the guide sequence comprises between 0-3 ribonucleotides that are not complementary to the predetermined sequence in the HBV genome. The mismatches are preferably located at the 5' end of the guide sequence.

The specific binding of a nucleic acid sequence can e.g. be achieved through complementary base pairing. It is, thus, preferred that the DNA-binding molecule that is able to specifically bind to a predetermined sequence in the hepatitis B virus genome comprises a DNA-binding sequence that is complementary to the predetermined sequence in the hepatitis B virus genome. The DNA-binding molecule comprises preferably a DNA-binding nucleic acid sequence (such as a guide RNA sequence) that is at least 70%, at least 80%, at least 90% or 100 % identical to the complement of the predetermined sequence in the hepatitis B virus genome. More preferably, the DNA-binding molecule comprises a DNA-binding nucleic acid sequence (such as a guide RNA sequence) that is from about 85% to 100% identical to the complement of the predetermined sequence in the hepatitis B virus genome.

In another embodiment, the DNA-binding molecule comprises a DNA-binding polypeptide sequence, preferably a polypeptide sequence derived from a zinc finger polypeptide, or from a transcription activator-like effector and functional variants thereof. For example, sequence variants may be used having one or more substitutions, insertions or deletions of amino acids relative to the sequence of a known zinc finger polypeptide, or from a transcription activator-like effector. However, polypeptide sequences derived from a zinc finger polypeptide, or from a transcription activator-like effector are characterized in that they retain the DNA-binding activity of the aforementioned polypeptide sequences.

The specific binding of a DNA-binding polypeptide sequence in the context of the present invention is a specific binding to the nucleic acid bases in the predetermined sequence in the HBV genome. Protein structures that bind specifically to nucleotide bases and, thus, provide a base pair-like binding mode are known in the art and described herein. The DNA-binding polypeptide sequence that is able to specifically bind to a sequence in the hepatitis B virus genome is preferably derived from a zinc finger polypeptide, or from a transcription activator-like effector.

Thus, in one embodiment, the DNA-binding molecule comprises a polypeptide sequence derived from a zinc finger polypeptide, such as a zinc-finger motif. Zinc-finger motifs (ZFs) are derived from natural transcription factors. Each finger is relatively small (~30 amino acids) and has one alpha-helix and two beta-strands (Carroll, 2014, Annu Rev Biochem, 83:14.1-14.31). A finger interacts with DNA through contacts between specific protein side chains and functional groups in the major groove. Usually, each finger binds 3 base pairs. Adequate binding specificity is provided when at least 3 consecutive fingers are used. Thus, in this embodiment, the DNA-binding polypeptide sequence that is able to specifically bind to a predetermined sequence in the HBV genome comprises at least 3 zinc-finger motifs. Suitable methods for the design of specific zinc-finger motifs are known in the art (e.g. described by Carrol, ibidem).

In a further embodiment, the DNA-binding molecule comprises a polypeptide sequence derived from a transcription activator-like effector (TALE). TALEs have a DNA-binding domain made up of tandem repeats of ~34 amino acids. Generally, the sequence of TALEs is highly conserved with the exception of residues in positions 12 and 13 (repeat variable di-residues) which vary according to the base pairs in the target sequence. Suitable methods for the identification of specific TALEs are known in the art (e.g. described by Carrol, ibidem). The DNA-binding activity of TALEs can be combined with a nuclease, such as *Fok*I*,* by fusion of the peptide sequences of TALE and nuclease, i.e. by expression as a fusion protein.

The predetermined sequence in the hepatitis B virus genome can be a part of the sequence encoding the X or S protein of the hepatitis B virus. Both, X and S protein are highly relevant for the generation of functional, i.e. infective, HBV particles from the HBV genome and/or the induction of tumors of the liver. Therefore, in one embodiment, the predetermined sequence in the hepatitis B virus genome is a part of the sequence encoding the X protein of the hepatitis B virus. In another embodiment, the predetermined sequence in the hepatitis B virus genome is a part of the sequence encoding the S protein of the hepatitis B virus.

The predetermined sequence in the hepatitis B virus genome is preferably a sequence that is conserved within a hepatitis B genotype and, preferably, conserved between different hepatitis B genotypes. It is particularly preferred that the predetermined sequence in the hepatitis B virus genome is conserved within hepatitis B genotype A, more preferably between hepatitis B genotypes A and D. The HBV genome is subject to mutations leading to variability in the genome sequences of different HBV strains. Nevertheless, the present invention identifies regions in the HBV genome that are conserved among different HBV genotypes/strains. Targeting of these sequences with the DNA-binding molecules of the present invention allows the reliable disruption of various HBV strains and, thus, omits the step of sequencing a new strain and subsequently designing a new DNA binding molecule. Specifically, two conserved regions in the HBV genome were identified. The conserved regions are in the ORF encoding the HBV S-Protein and in the ORF encoding the HBV X-Protein, and are shown in SEQ ID NOs:19 and 20, respectively. In one embodiment, the conserved predetermined sequence of the hepatitis B virus genome is a part of the region spanning nucleotide positions 190 to 262 of the HBV genome (SEQ ID NO:19), i.e. nucleotide positions 32 to 105 of the ORF encoding the HBV S-Protein. However, it is also possible that the conserved predetermined sequence of the hepatitis B virus genome has a sequence that is at least 70%, 80%, 90% or 95% identical to a part of the region spanning nucleotide positions 190 to 262 of the HBV genome (SEQ ID NO:19), such as a sequence that comprises at least 1, 2, 3, 4, 5, 6, 7, 8, 9 or 10 mismatches relative to SEQ ID NO:19. In another embodiment, the conserved predetermined sequence of the hepatitis B virus genome is a part in the region spanning nucleotide positions 1520 to 1576 of the HBV genome (SEQ ID NO:20), i.e. nucleotide positions 142 to 200 of the ORF encoding the HBV X-Protein. However, it is also possible that the conserved predetermined sequence of the hepatitis B virus genome has a sequence that is at least 70%, 80%, 90% or 95% identical to a part of the region spanning nucleotide positions 1520 to 1576 of the HBV genome (SEQ ID NO:20), such as a sequence that comprises at least 1, 2, 3, 4, 5, 6, 7, 8, 9 or 10 mismatches relative to SEQ ID NO:20. The start point of the HBV genome is considered the EcoRI restriction site, the reference sequence of the HBV genome is NCBI: GenBank: X02763.1. Thus, preferably, the predetermined nucleotide sequence within the hepatitis B virus genome is part of the sequence encoding the X or S protein of the hepatitis B virus.

Preferred predetermined sequences from the conserved region in the ORF encoding the S-Protein are e.g. the sequences from position 1 to 20 of SEQ ID NOs:13 and 14 (comprising the predetermined sequence at positions 1-20 and a PAM motif at positions 21-23), or variants or complements thereof. Preferred predetermined sequences from the conserved region in the ORF encoding the X-Protein are e.g. the sequences from position 1 to 20 of SEQ ID NOs: 15 and 16 (comprising the predetermined sequence at positions 1-20 and a PAM motif at positions 21-23), 1-23), or variants or complements thereof. For example, sequence variants may have one or more (e.g. 1, 2, 3) substitutions, insertions or deletions of nucleotides relative to the sequences of SEQ ID NOs: 13-16 or their complements. The variant will have at least 85% sequence identity to the sequence of SEQ ID NOs: 13-16 or their complements, preferably at least 86, 87, 88, 89, 90, 91, 92, 93, 94, or 95% sequence identity. In a particularly preferred aspect of the invention, the predetermined sequence consists of the sequence of SEQ ID NO:13, 14, 15 and/or 16. The predetermined sequence of the hepatitis B virus genome may have a length of 5-50, 8-30, 10-25 nucleotides. Preferably, the sequence has a length of about 10, about 15, about 20, about 25 nucleotides. Most preferably, the sequence has a length of about 20 nucleotides. This includes sequences having a length of 16, 17, 18, 19, 20, 21, 22, 23, and 24 nucleotides. Thus, the predetermined nucleotide sequence within the hepatitis B virus genome can also comprise or consist of 17 to 20 consecutive nucleotides of positions 1-20 of any of SEQ ID NO: 13, 14, 15, or 16, or variants or complements thereof.

Said predetermined sequence is a part of the hepatitis B virus genome. Infectious viral HBV particles comprise a circular DNA that is enclosed in an outer lipid envelope (containing the proteins M, S and L) and an icosahedral nucleocapsid protein core. After infection of a cell, the HBV establishes its genome as episomal covalently closed circular (ccc) DNA and occasionally integrates into the host cell chromosomes. (Jiang, 2012, Genome Res. 22(4):593-601). In all of these forms, i.e. in circular, cccDNA and chromosomally integrated form and possible intermediates, the HBV genome can be disrupted by the DNA-binding molecules for use according to the present invention. Thus, the HBV genome may be in circular, episomal cccDNA and/or chromosomally integrated form.

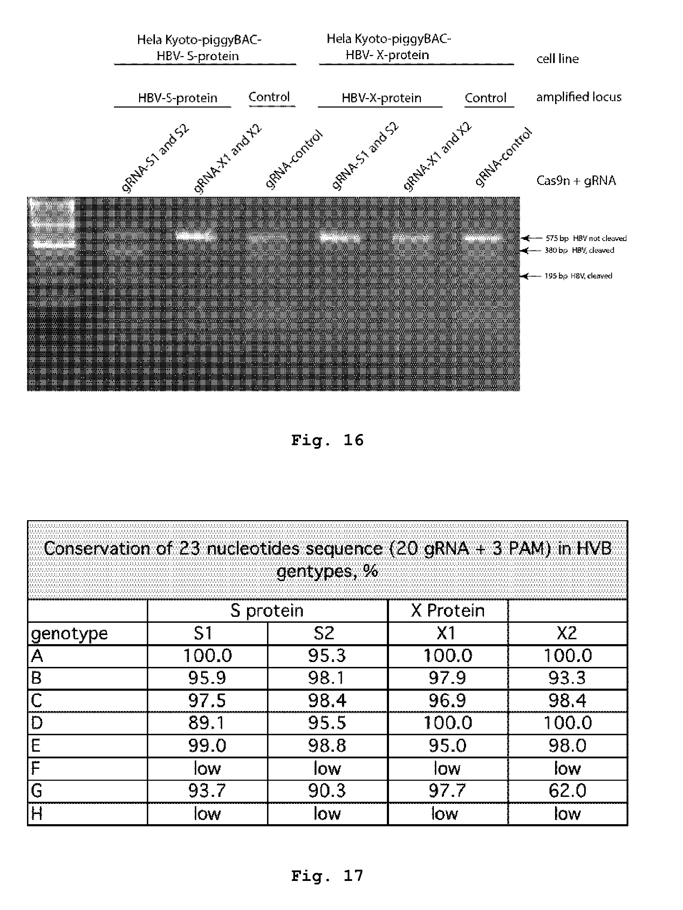

The term "hepatitis B virus" includes the different HBV genotypes and serotypes known in the art. There are nine different genotypes (A-I) as well as numerous subgenotypes, with different distribution in different geographic regions. The genotypes A2 and D are most predominant in Europe. Thus, HBV can have any of genotypes A through I. Genotypes E-H are rare. Therefore, it is particularly preferred that the predetermined sequence of the HBV genome is conserved in HBV genotypes A through D or I. The genomes of all presently known genotypes of HBV can be inactivated according to the present invention. The sequences in the X and S protein targeted in the experiments elsewhere described herein are conserved in the genotypes A, B, C and E (>95% of the strain variants contain these sequences), and in genotype D with 89% conservation for the target in protein S and 100% conservation for target in protein X (Fig. 17). Therefore, it is particularly preferred that the predetermined sequence of the HBV genome is conserved in HBV genotypes A through E, more preferably in genotypes A, B, C and E. In consequence, the HBV genome is preferably a HBV genotype A, B, C, D or E, more preferably genotype A, B, C or E. In the HBV genotype A genome these targeted sequences have a conservation of >95% (protein S sequences) and 100% (protein X sequences). In consequence, it is particularly preferred that the HBV genome is a HBV genotype A genome. An example of a genome from a HBV isolate of genotype A is shown in SEQ ID NO:21.

The DNA-binding molecule is for use in a method of treating and/or preventing hepatitis B and/or a hepatitis D virus infection in a patient. In a preferred embodiment, the DNA-binding molecule is for use in a method of treating and/or preventing hepatitis B virus infection in a patient. As outlined above, hepatitis D virus can only assemble as an infectious virus particle when it is supplemented with the HBsAg envelope of the HBV. Thus, the present invention is also useful against hepatitis D virus infection. Thus, in another embodiment, the DNA-binding molecule is for use in a method of treating and/or preventing hepatitis D virus infection in a patient. Naturally, the DNA-binding molecule can also be used in a method of simultaneously treating or preventing a hepatitis B virus and a hepatitis D virus infection in a patient.

The patient is preferably a patient in need of anti-HBV and/or anti-HDV treatment. Thus, the patient usually has a HBV and/or a HDV infection. Nevertheless, the present DNA-binding molecules are also suitable for use in the prevention of a HBV and/or a HDV infection. Therefore, the patient can also be from a group in the population for which the risk of a future infection is increased, such as persons working in health care, social workers, drug addicts, persons receiving (blood) transfusions, persons with a suppressed immune system and persons with a promiscuous life style.

An infection with hepatitis B virus and/or a hepatitis D virus infection can be chronic or acute. Furthermore, the infection can also be silent. Methods for the diagnosis of such infections are well known in the art. For example, serological assays to detect specific HBV antigens such as the HBsAg (Hepatitis B surface antigen) and HBc (Hepatitis B Virus core antigen) in the blood, nucleic acid assays to detect and quantify HBV DNA, such as PCR and real-time PCR analysis of patients liver samples can be used in the diagnosis (Valsamakis, 2007, Clin. Mi-crobiol. Rev. 20(3):426). As outlined before, HBV and HDV establish their genomes in human liver cells/hepatocytes. Thus, the DNA-binding molecule according to the present invention is preferably used for the treatment or prevention of hepatitis B virus and/or a hepatitis D virus infection in the liver of a patient.

The method of treating and/or preventing hepatitis B and/or a hepatitis D virus infection comprises the administration of at least one DNA-binding molecule in combination with a nuclease. Administration of at least one DNA-binding molecule means that one or more than one different DNA-binding molecules, such as 1, 2, 3, 4, 5, 6, or more different DNA-binding molecules are administered. In one embodiment, one DNA-binding molecule is administered. In a preferred embodiment, two different DNA-binding molecules, such as two sgRNAs, are administered in combination with a nuclease. Different DNA-binding molecules are able to bind to different predetermined sequences in the HBV genome. In a preferred embodiment, the two different DNA binding molecules bind specifically to the predetermined sequences SEQ ID NO:13 and 14, respectively. In a further preferred embodiment, the two different DNA binding molecules bind specifically to the predetermined sequences SEQ ID NO:15 and 16, respectively.

It is particularly preferred that two sgRNAs that are able to bind specifically to the predetermined sequences SEQ ID NO:13 and 14, respectively, are administered in combination with the nuclease Cas9n (which is described herein below). It is further preferred that two sgRNAs that are able to bind specifically to the predetermined sequences SEQ ID NO:15 and 16, respectively, are administered in combination with the nuclease Cas9n.

Thus, the at least one DNA-binding molecule is administered in combination with a nuclease. Administration of DNA-binding molecule and nuclease to the patient can, thus, be at the same time or directly after one another, e.g. within a period of less than 6 hours, less than 2 hours, less than 1 hour, less than 30 min, or less than 10 min. Preferably, the DNA-binding molecule and the nuclease are mixed before administration. Suitable routes of administration are described elsewhere herein. The DNA-binding molecule and/or the nuclease can also be administered in the form of a vector or vectors that encode the DNA-binding molecule and/or the nuclease. Suitable vectors are described elsewhere herein.

It has been shown in the art that periodically repeated administration of anti-HBV medicaments increases the success rate of eradication of viral genomes considerably. Therefore, in one embodiment, the administration of DNA-binding molecule and nuclease is repeated, e.g. approximately monthly or every year.

Administration will be in an amount of the DNA-binding molecule that is effective to reduce the amount of intact HBV genomes in the patient. The therapeutically effective amount of the DNA-binding molecule or the vector encoding the same to be administered will depend on several parameters, such as the mode of administration, the severity of the infection, the history of the disease, the age, height, weight, health, and physical condition of the individual to be treated, and the like. A therapeutically effective amount of the DNA-binding molecule or the vector encoding the same can be determined by one of ordinary skill in the art without undue experimentation given the disclosure set forth herein.

As the HBV genome is a DNA genome, the nuclease is a deoxyribonuclease. The nuclease may introduce single- or double strand breaks (such as blunt or sticky ended breaks). Double strand breaks are often repaired by the cell through homologous recombination (HR), where cells are using an unbroken sister chromatid or homologous chromosome as template. Additionally, double strand breaks can also be repaired by non-homologous end joining (NHEJ), wherein broken ends are rejoined without regard for homology and the products often contain small insertions or deletions (of up to several 100 bp), which cause shifts of the reading frame. In the absence of an editing template cells typically repair the dsDNA break by NHEJ. Should no mutation occur during repair of the break, the DNA binding molecule would again guide the nuclease to the predetermined sequence in the HBV genome.

The nuclease is able to cleave in or adjacent to the predetermined nucleotide sequence within the HBV genome. "Adjacent" in this context is to be understood as a distance of between 0 and 35 nt. Preferably, the nuclease can perform cleavage of substantially any DNA sequence to which it is directed, i.e. the nuclease does not need to possess sequence-specificity. When such a sequence unspecific nuclease is used in the context of the preset invention, cleavage specificity can be achieved by the invention through the use of specific DNA-binding molecules.

For example, the nuclease may be selected from the group consisting of Type II restriction endonucleases, Type IIS endonucleases, such as the FokI cleavage domain, Cas protein, such as Cas9 protein, a Cas9-derived nickase (Cas9n, such as a Cas9n shown in SEQ ID NOs:22 and 24) and functional variants thereof. The Cas protein may be from a Type II CRISPR/Cas system. The Cas protein is derived from a CRISPR-Cas system that targets DNA, i.e. preferably not from Type IIIB CRISPR/Cas system.

Type II restriction endonucleases recognize a short DNA sequence and cut within it. Type IIS endonucleases, such as the FokI cleavage domain, cut some distance from their recognition site. Both types of nucleases as are well known in the art. For example, Type IIS endonucleases are comprised in zinc finger nucleases (ZFN) and in Transcription activator-like effector nucleases (TALENs). Further, the nuclease may also be a variant of a Type II restriction endonuclease or a Type IIS restriction endonuclease that has been modified to introduce a single strand DNA break instead of a double strand break. Such variants are described in the art, e.g. by Carroll (2014, Annu Rev Biochem, 83:14.1-14.31).

Cas9 protein is the CRISPR associated endonuclease that catalyzes blunt-ended dsDNA breaks by introducing two separate single stranded DNA nicks, mediated by HNH and RuvC nuclease domains. In the original bacterial CRISPR systems Cas9 is guided by the crRNA/tracrRNA complex and has 2 nuclease active sites, one to cleave each strand of the target DNA. It, thus, introduces a double strand break. In one embodiment of the invention, thus, the nuclease is a Cas9 nuclease or a functional variant or fragment thereof. Cas9 does not require a specific target or recognition sequence other than the PAM motif described below and is expected to cleave any DNA sequence to which it is directed.

Cas9 requires a very short protospacer adjacent motif (PAM) in the DNA immediately downstream of the target region. A PAM is a targeting component that also serves as a self versus non-self recognition system to prevent the crisper locus itself from being targeted. Preferably, a PAM is immediately downstream of (i.e. 3' of) the predetermined nucleotide sequence within the hepatitis B virus genome. "Immediately" in this context means that between the predetermined nucleotide sequence and the PAM in the HBV genome are preferably no further nucleotides.

Many CRISPR-Cas systems have different PAM requirements which are known in the art. PAMs usually have a length from 3 to 10 nucleotides, depending on the nuclease used. For example, Cas9 of *Streptococcus pyogenes* requires a PAM with the sequence NGG or NAG, wherein N is any nucleotide (Mali et al., 2013, Nature Methods, 10(10):957-963; Terns and Terns, 2014, Trends Genet, 30(3):111-118). Therefore, in one embodiment, the nuclease is Cas9 *Streptococcus pyogenes* or a functional fragment or variant thereof and the PAM has the sequence NGG, wherein N is any nucleotide. In another embodiment, the nuclease is Cas9 of *Neisseria meningitidus* or a functional variant thereof and the PAM has the sequence NNNNGA/CTT (Hou et al., 2013, PNAS, 110:15644-49). In another embodiment, the nuclease is Cas9 of *Streptococcus thermophilus* or a functional variant thereof and the PAM has the sequence NNAGAAW (Terns and Terns, ibidem). In another embodiment, the nuclease is Cas9 of *Treponema denticola* or a functional variant thereof and the PAM has the sequence NAAAAN (Terns and Terns, ibidem). N is any nucleotide.

A functional variant of the above nucleases is a nuclease that has been derived from the above nucleases by deletion, insertion or mutation of one or more amino acids in the nuclease. In the present context, the term "variant" should be understood as a nuclease which is functionally equivalent to one of the above nucleases, e.g. has substantially the same, or improved, properties e.g. regarding its nuclease activity. Such variants, which may be identified using appropriate screening techniques, are a part of the present invention.

In the context of the present invention, the term "functional variant" also includes variants that introduce a single strand break instead of a double strand break (so-called nickases). Several groups have made derivatives of Cas9 that cut only one strand at the target (a reference to the respective publications can be found in Carroll, 2014, Annu Rev Biochem, 83:14.1-14.31). Functional variants of Cas9 which introduce a single strand break instead of a double strand break are termed Cas9 nickases (Cas9n). Obligate heterodimer modifications of the FokI cleavage domain can also be used in the context of the present invention.

Thus, in a further embodiment, the nuclease is a Cas9-derived nickase (Cas9n), such as the Cas9n described by Mali et al. (2013, Nature Biotechnol., 31:833-38), by Cho et al. (2013, Genome Res, 24:132-41 or by Ran et al. (2013, Cell, 89). In Cas9n either HNH or RuvC domain nuclease activity is inactivated, such as e.g. by mutating one or more amino acids in the in the nuclease domain. Thus, Cas9n comprises only one nuclease active site. The person skilled in the art is able to identify suitable inactivating mutations and to generate mutated sequences. Preferably, the nuclease is Cas9n wherein the RuvC domain is inactivated, such as *Streptococcus pyogenes* Cas9 D10A mutant that is shown in SEQ ID NO:22 (described by Ran et al. ibid), e.g. encoded by SEQ ID NO:23. However, the nuclease may also be Cas9n wherein the HNH domain is inactivated, such as *Streptococcus pyogenes* Cas9 H840A mutant.

If the nuclease is a Cas9n, two sgRNAs are used to direct Cas9n molecules simultaneously to a pair of sequences near each other on opposite strands of the HBV genome. Both sites are nicked and the resulting double strand break induces both NHEJ and HR. This way, nickases, such as Cas9n, provide an even better specificity than the original nuclease, because two DNA-binding molecule/nuclease (e.g. sgRNA-Cas9n) complexes are required for activity, i.e. cleavage and disruption of a DNA double strand (Mali et al., 2013, Nature Methods, 10(10):957-963). Because the majority of nicks do not result in NHEJ events, only the coordinate nicks at the targeted site will initiate genome-editing events.

Thus, in a preferred embodiment, the method of the present invention comprises the administration of two DNA binding molecules in combination with a nuclease that introduces single strand breaks, such as a Cas9 derived nickase (Cas9n). It is particularly preferred that the two DNA binding molecules are sgRNA and the nickase is a Cas9n. In this embodiment, the two DNA binding molecules are able to bind specifically to two predetermined nucleotide sequences, which are near each other on opposite strands of the hepatitis B virus genome. In one embodiment, the two DNA binding molecules are able to bind specifically to the predetermined sequence shown in positions 1-20 of SEQ ID NO: 13 and SEQ ID NO: 14, respectively, or to the respective complementary sequences. In another embodiment, the two DNA binding molecules are able to bind specifically to the predetermined sequence shown in position 1-20 of SEQ ID NO: 15 and SEQ ID NO: 16, respectively, or to the respective complementary sequences. However, the two DNA binding molecules can also be able to bind specifically to variants of these predetermined sequences that share at least 70%, 75%, 80%, 85%, 90%, 95% or 100 % sequence identity to any of SEQ ID NO: 13-16.

Two sequences are considered to be "near" each other when not more than 100, 95, 90, 85, 80, 75, 70, 65, 60, 55, 50, 45, 40, 35 or 30 nucleotides are between them.

Functional variants of the above cases also include nucleases that are human-codon-optimized, such as the Cas9n protein of SEQ ID NO:22, that is e.g. encoded by SEQ ID NO:23. The CRISPR/Cas systems employed herein are originally bacterial defense systems. As different organisms often show particular preferences for one of the several codons that encode the same amino acid, the translation efficiency of a nuclease-encoding vector which is administered to the patient is increased when the original bacterial codons are at least partially exchanged with codons preferred in mammalian cells. Methods for the design and the cloning of codon-optimized nucleases are well known in the art.

It is evident that the functional variants of the nucleases described herein included proteins that comprise further useful sequence motifs, such as nuclear localization signals or tags, such as His-tags. Thus, in one embodiment, the nuclease comprises at least one NLS. NLS are well known in the art. An exemplary nuclease that can be used in the context of the present invention comprising a NLS at the N and at the C terminus and a His-tag at the C terminus is the NLS-His-Cas9 D10A-His nickase shown in SEQ ID NO:24, that is e.g. encoded by SEQ ID NO:25.

The nuclease is guided by the DNA-binding molecule to said predetermined nucleotide sequence where it cleaves the DNA to disrupt the viral genome. For this purpose, the DNA-binding molecule binds or is bound to the nuclease thereby directing the nuclease to the predetermined sequence in the HBV genome to which the DNA-binding molecule is able to bind. Consequently, in a preferred embodiment, the DNA-binding molecule is bound or is able to bind to the nuclease. Thus, the nuclease and the at least one DNA-binding molecule may be comprised in a single molecule or in separate molecules. The nuclease binds or is bound to the at least one DNA-binding molecule by forces that are sufficiently strong to prevent dissimilation of the nuclease from the at least one DNA-binding molecule under physiological conditions, such as cell culture conditions or conditions in the human or animal body. Such forces include covalent, ionic and van-der-Waals bonds and the like. Thus, the bond between the nuclease and the at least one DNA-binding molecule is stable under physiological conditions. Physiological conditions are e.g. a temperature in the range of 20-40 degrees Celsius at a pH of 6-8. In one embodiment of the invention, the DNA binding molecule and nuclease are comprised in a single molecule. In this embodiment, for example, the nuclease is a Type IIS endonuclease, such as the FokI cleavage domain, and the DNA-binding molecule comprises zinc finger motives. Alternatively, in this embodiment, the nuclease is a Type IIS endonuclease, such as the FokI cleavage domain, and the DNA-binding molecule comprises a Transcription activator-like effector (TALE). In another embodiment of the invention, the DNA binding molecule and nuclease are two separate molecules that are not connected by covalent bonds. In this embodiment, the DNA binding molecule further comprises a nuclease binding site. For example, the nuclease Cas9 or variants thereof may e.g. be bound by the crRNA/tracrRNA hybrid section that is comprised in sgRNA. Thus, in this embodiment, for example, the nuclease is a CRISPR/Cas nuclease, such as Cas9 or Cas9n, and the DNA-binding molecule is a single guide RNA.

The nuclease cleaves the DNA to disrupt the viral genome. By disruption, the viral genome is inactivated. As outlined elsewhere herein, the cleaving of the DNA can be introduction of a single or a double strand break by the nuclease. A HBV genome is considered to be disrupted when the generation of functional, i.e. infective, HBV particles from this genome and/or the induction of tumors of the liver is no longer possible. For example, when the S gene of HBV is disrupted no infectious HBV particles will be produced. When the X gene of HBV is disrupted the produced HBV particles will no longer be able to induce tumors of the liver due to a lack of the functional X protein. As described elsewhere herein, disruption of the viral genome is achieved by the present invention through the introduction of DNA strand breaks and thereby induced endogenous error prone DNA repair mechanisms that lead to deletions, insertions and/or frame shift mutations. According to the invention a double strand break or two adjacent single strand breaks are introduced in opposite DNA strands. The generation of functional HBV particles may, e.g., be prevented by inhibition of the replication of the HBV DNA and/or the inhibition of synthesis of one or more of the functional HBV proteins. Thus, in one embodiment, the disrupted genome comprises at least one deletion, insertion and/or frame shift mutation that renders at least one of the HBV proteins functionally inactive or fully prevents the expression of at least one of the HBV proteins. The HBV protein is preferably selected from the group consisting of X and S protein.

### Oligoribonucleotide

In another aspect, the invention relates to an oligoribonucleotide comprising a sequence set forth in SEQ ID NOs:9-12 or variants or complements thereof. SEQ ID NOs:9-12 are gRNA sequences identified in the examples described herein below.

A variant of a guide sequence in the sense of the present invention is a functional variant. A variant has been derived from the above guide sequences by deletion, insertion or mutation of one or more nucleotides. In the present context, the term "variant of a guide sequence" should be understood as an oligoribonucleotide sequence which is functionally equivalent to one of the above sequences, e.g. has substantially the same, or improved, properties e.g. regarding its specific binding to the predetermined sequence of the HBV genome. Such variants, which may be identified using appropriate screening techniques, are a part of the present invention.

The oligoribonucleotide (i.e. the RNA molecule) is preferably a synthetic guide RNA (sgRNA) as described elsewhere herein. Then, the oligoribonucleotide can be used in a CRISPR system as described above. Further, in this embodiment, the oligoribonucleotide further comprises a crRNA and/or a tracrRNA sequence, preferably a crRNA/tracrRNA hybrid sequence as described elsewhere herein.

The oligoribonucleotide has a length of less than 500, preferably less than 300, more preferably less than 200, more preferably less than 150 ribonucleotides. At present sgRNAs exists that are as short as 87 ribonucleotides, comprising 20 ribonucleotides guide sequence and crRNA/tracrRNA. Thus, the oligoribonucleotide may have a length of from 80 to 500, preferably from 85 to 300, more preferably from 87 to 160 ribonucleotides. For example, the oligoribonucleotide can have a length of approximately 100 ribonucleotides, such as 103 ribonucleotides.

The sequence that is able to bind specifically to a predetermined sequence of the hepatitis B virus genome has a length of at least at least 16, 17, 18, 19 or 20 nucleotides. The sequence is a guide sequence as described elsewhere herein.

### Vector

In a further aspect, the invention relates to a vector encoding a DNA-binding molecule or an oligoribonucleotide of the present invention. The vector may further encode a nuclease as described herein. However, the nuclease may also be encoded on a vector that is administered separately or administered in protein form directly.

As used herein, a vector refers to a viral or polynucleotide construct (i.e. in other words, a viral or non-viral vector), wherein the polynucleotide construct is normally comprised of DNA or mRNA. The vector allows for the genetic transfer, replication and expression of an inserted polynucleotide in a host cell, wherein said polynucleotide is not native to said host cell, or which is native to said host cell, but has been modified. Replication of the vector can be achieved either by integration of the vector into the genome of the host cell, or by episomal replication (e.g. plasmids). In the latter case, the vector comprises a functional origin of replication which allows the replication of the plasmid in the respective host cell.

In one embodiment the vector comprises one or more DNA sequences encoding sgRNA as described elsewhere herein, wherein the one or more DNA sequences are selected from the group consisting of SEQ ID NO:5-8. For example, the vector may comprise two DNA sequences encoding sgRNAs S1 and S2 shown in SEQ ID NO:5 and 6, respectively. The vector may also comprise two DNA sequences encoding sgRNAs X1 and X2 shown in SEQ ID NO:7 and 8, respectively. Alternatively, said sgRNAs may be encoded by two different vectors. In a further embodiment, the vector comprises additionally a sequence encoding a nuclease, such as a sequence encoding a Cas9n selected from the group consisting of SEQ ID NOs: 22 and 24.

The vector may contain further regulatory elements, such as promoters driving the expression of the encoded DNA-binding molecule, oligoribonucleotide and/or nuclease. The sequence encoding the DNA-binding molecule, oligoribonucleotide and/or nuclease may be operably linked to a promoter element and, optionally, to an enhancer element. A suitable promoter is, e.g., the immediate early promoter of the cytomegalovirus (CMV). A suitable promoter is also the chicken beta-actin human codon optimized promoter (CBh). The promoters and enhancers may further be selected from those known in the art, such as the promoters and enhancers of the LTR of Rous sarcoma virus, the TK gene of HSV-1, the early or late promoters of SV40, the adenovirus major late promoter (MLP), phosphoglycerate kinase genes, metallothionein genes, a-1 antitrypsin genes, albumin genes, collagenase genes, elastase I genes, β-actin genes, β-globin genes, γ-globin genes, α-fetoprotein genes, and muscle creatin kinase genes. When the DNA binding molecule is a sgRNA, then the vector may further comprise a RNA polymerase III (pol III) promoter, such as the U6 promoter, which is operably linked to the sequence encoding the sgRNA. The expression vector also optionally comprises transcription termination sequences and polyadenylation sequences, such as a bovine growth hormone polyadenylation signal (bGHpolyA), for improved expression of the nuclease. Suitable transcription terminator and polyadenylation signals can, for example, be derived from SV40 (Sambrook et al (1989), Molecular Cloning: A Laboratory Manual). Any other element which is known in the art to support efficient expression may be added to the vector, such as the Woodchuck post-transcriptional regulatory element (wPRE).

The DNA-binding polypeptide or the nuclease encoded by the vector may additionally comprise nuclear localization signals (NLS) to facilitate transport of the expression product to the nucleus. Suitable NLS are well known in the art.

The vector may further comprise genes encoding one or more markers that are coupled to the expression of DNA-binding molecule, oligoribonucleotide and/or nuclease. Suitable marker genes are e.g. genes encoding fluorescent proteins, such as red fluorescent protein (RFP) or (enhanced) green fluorescent protein (GFP). RFP can e.g. be amplified from pDSV-mRFP (Tonelli et al., Biotechniques, 2006, 41). In one embodiment a marker gene, such as a gene encoding GFP may be cloned 3' of the sequence encoding the DNA binding molecule, oligoribonucleotide and/or nuclease described herein, wherein the gene encoding GFP preferably lacks a start codon ATG and preferably is in another reading frame than the sequence encoding the DNA binding molecule, oligoribonucleotide and/or nuclease.

In one embodiment, the vector is a non-viral vector, such as a plasmid. Suitable non-viral vectors are known in the art. Examples for non viral vectors suitable for protein and RNA expression in mammalian cells comprise, for example, the vectors pEGFP-neo-GFP (Clontech), pEGFP-N1 (Clontech) and pX355. In a preferred embodiment, the vector is a pX355 plasmid comprising a pol III promoter (U6) driving expression of a sgRNA, a chicken beta-actin human codon optimized promoter (CBh), a gene encoding Cas9 nickase (human codon optimized, D10A mutant) with two nuclear localization signals (NLS) (SEQ ID NO:23) and a bovine growth hormone polyadenylation signal (bGHpolyA) (Ran et. al., 2013, Cell).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus and adeno-associated virus (AAV), orthomyxovirus, paramyxovirus, papovavirus, picornavirus, or alphavirus. For an overview of the different viral vector systems, see Nienhuis et al., Hematology, Vol. 16: Viruses and Bone Marrow, N.S. Young (ed.), 353-414 (1993). Viral vectors such as AAV have been developed for delivery to the human liver and have successfully been used in human clinical trials for metabolic disorders (Nathwani et al., 2011, NEJM, 365:2357-65). Thus, in a preferred embodiment, the vector is an adeno-associated viral vector. Where the subject to be treated is a mammal, such as a human, the vector to be administered typically is a viral vector.

The viral or non-viral vector may be transformed, transfected or transduced into the host cell or organism by any suitable method. For example, non-viral vectors may be transferred into target cells for example by the lipofection method, calcium-phosphate co-precipitation method, DEAE-dextran method and direct DNA introduction methods using micro-glass tubes and the like, as described e.g. in Maniatis et al. 1982, Molecular Cloning, A laboratory Manual, Cold Spring Harbor Laboratory.

Typically, viral vectors are able to infect the target cells and introduce the gene of interest into the cell. The viral vectors may either be endogenously able to infect the target cell or be engineered to do so. Such engineering may be that the viral vector contains a ligand molecule in its viral capsid and/or envelope which binds to surface molecules of the target cells.

### Pharmaceutical compositions

The DNA-binding molecule, the oligoribonucleotide or the vector of the present invention will normally be provided in the form of a pharmaceutical composition which also comprises one or more excipients, carriers and/or diluents which are suitable for the intended way of administration. Generally, the compound may be administered in any suitable form that does not interfere with its HBV genome-disruptive activity. The DNA-binding molecule, oligoribonucleotide or vector may be administered orally in the form of tablets, capsules, granule, powder, liquids, and the like. Alternatively, the DNA-binding molecule, oligoribonucleotide or vector may be formulated for being administered parenterally, e.g. by intravenous injection or intravenous infusion. Upon intravenous delivery, the DNA-binding molecule, oligoribonucleotide or vector will disperse to target sites randomly. The entry into target cells such as the liver cells/hepatocytes can be ensured by utilising vectors that are engineered to preferentially bind specific cell surface receptors, such as sodium taurocholate cotransporting polypeptide, which are e.g. specific to HBV target cells (Yan et al., 2012, elife, 1: e00049). In a preferred aspect, administration is to the subject by intravenous infusion, more preferably by short-term infusion within less than 60 min, e.g. within 30 min, 20 min or 15 min.

Apart from the DNA-binding molecule, the oligoribonucleotide or the vector, the pharmaceutical composition provided by the present invention may further comprise other compounds which are commonly used in the treatment of HBV or HDV infections, such as interferon alpha, pegylated interferon-α, nucleoside or nucleotide analogues.

### Kit

In yet another aspect, the invention is concerned with a kit comprising a1) at least one DNA-binding molecule which is able to specifically bind to a predetermined nucleotide sequence within the hepatitis B virus genome; or a2) at least one oligoribonucleotide comprising a sequence set forth in SEQ ID NOs:9-12 or variants or complements thereof; and b) at least one nuclease which is guided by the DNA-binding molecule to said predetermined nucleotide sequence where it cleaves the DNA to disrupt the hepatitis B virus genome. The at least one DNA-binding molecule or the at least one oligoribonucleotide and the at least one nuclease are bound or are able to bind to each other. The DNA-binding molecule, the oligoribonucleotide and the nuclease are described elsewhere herein. The kit may also comprise said at least one DNA-binding molecule or said at least one oligoribonucleotide; and/or said nuclease in the form of a vector as described elsewhere herein.

The kit is preferably for use in a method of treating and/or preventing hepatitis B and/or a hepatitis D virus infection in a patient. However, the kit can of course also be used *in vitro* for the inactivation of a hepatitis B virus genome and/or a hepatitis D virus genome.

### Uses of the DNA-binding molecules defined herein

The invention further comprises use of a DNA-binding molecule which is able to specifically bind to a predetermined nucleotide sequence within the hepatitis B virus genome, which DNA-binding molecule is defined herein above, for the *in vitro* inactivation of a hepatitis B virus genome and/or a hepatitis D virus genome. In this use, a nuclease is guided by the DNA-binding molecule to said predetermined nucleotide sequence where it cleaves the DNA to disrupt the hepatitis B virus genome. Moreover, the use of a DNA-binding molecule as defined herein is comprised by the present invention, wherein said DNA-binding molecule comprises of a sequence set forth in SEQ ID NOs:9-12 or variants or complements thereof.

### Methods

In another aspect, the invention relates to an *in vitro* method for the disruption of a hepatitis B virus genome and/or a hepatitis D virus genome, comprising contacting the genome with (a) at least one DNA-binding molecule which is able to specifically bind to a predetermined nucleotide sequence within the hepatitis B virus genome, and (b) at least one nuclease which is guided by the DNA-binding molecule to said predetermined nucleotide sequence where it cleaves the DNA to disrupt / inactivate the hepatitis B virus genome.

Finally, the invention also relates to a method for treating and/or preventing hepatitis B in a patient, said method comprising administering to said patient (a) at least one DNA-binding molecule which is able to specifically bind to a predetermined nucleotide sequence within the hepatitis B virus genome; or (b) at least one nuclease which is guided by the DNA-binding molecule to said predetermined nucleotide sequence where it cleaves the DNA. The DNA-binding molecule and the nuclease are described elsewhere herein.

Administration may be, for example, by direct injection or non-invasive catheter or injector methods. Alternatively, target cells that have been removed from a subject, for example, by a biopsy procedure, may be transfected with the DNA binding molecule, oligoribonucleotide, nuclease and/or vector construct in an *ex vivo* procedure. The cells can then be implanted or otherwise administered into a subject, preferably into the subject from whom they were obtained.

### SHORT DESCRIPTION OF THE FIGURES

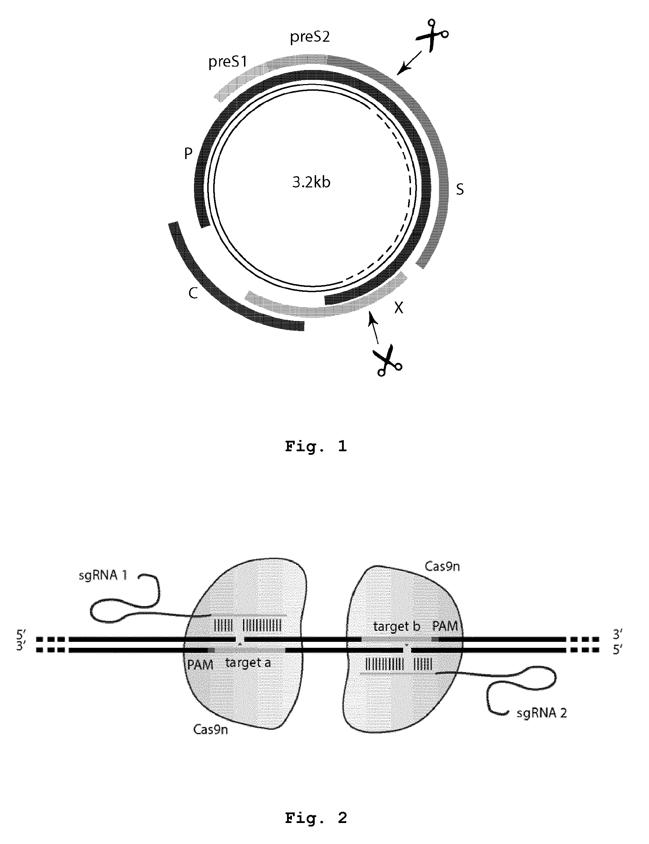

- **Figure 1**: is a schematic representation of the Hepatitis B virus genome. Relaxed circular DNA (rcDNA) of the HB virion (thin continuous line), which is converted to cccDNA (thin continuous and dotted line) following hepatocyte infection, is indicated in the center of the map. The four viral transcripts of the core (C), polymerase (P), surface (S) and X proteins are shown at the outside. Regions targeted by Cas9n via guide RNA (gRNA) specific to S and X proteins are indicated by arrows.
- **Figure 2**: is a schematic depiction of the CRSIPR-Cas9n approach to induce doublestranded DNA breaks utilizing Cas9-derived nickase (Cas9n) and a pair of sgRNA. Cas9n is guided for DNA binding by a pair of target-specific sgRNAs (sgRNA1 and sgRNA2) complementary to the DNA target. Cas9 D10A nickases bearing D10A mutation are employed to cleave only sequences in the strand complementary to the gRNA (target a and target b). A complex of two sgRNAs and two units of Cas9n protein enables the cleavage of the opposite strands of DNA creating a double stranded break. Target sequences are comprised of 20 bp recognized by a gRNA and NGG protospacer adjacent motif (PAM), immediately upstream of the 20 bp target.

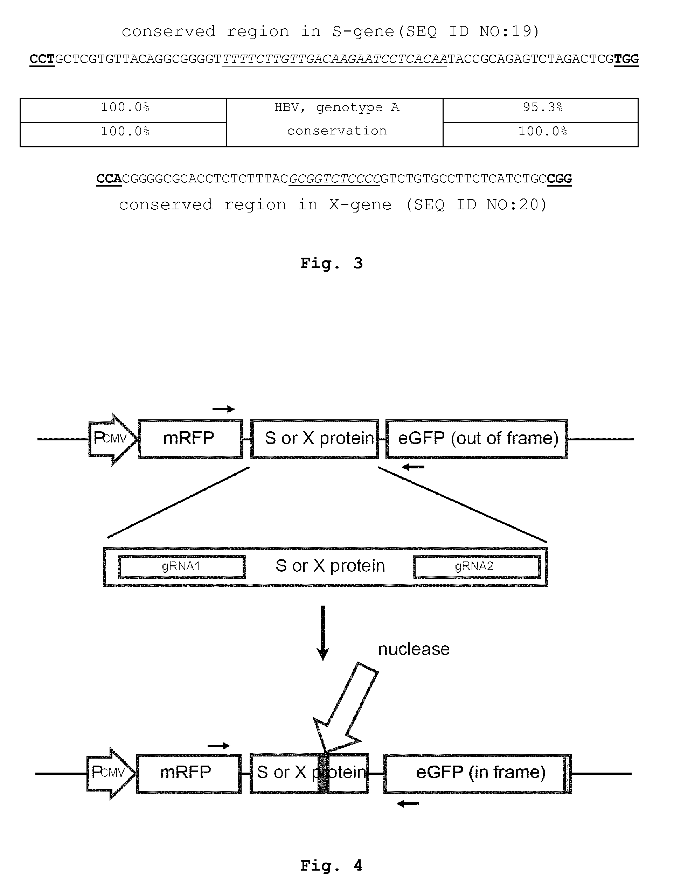

- **Figure 3**: shows the DNA sequence and sequence conservation of the targeted conserved regions within the ORFs of HBV S and X proteins (SEQ ID NOs:19 and 20, respectively). Target sequences of protein S and X are depicted, each encompassing proto-spacer adjacent motifs (PAM, bold and underlined), 2x20 nucleotides complementary to gRNA (normal letters) and offset distance between the two sequences complementary to gRNA (italic and underlined).
- **Figure 4**: shows a schematic depiction of the reporter plasmid for detection of Cas9n nuclease activity on HBV S or X target sequences. RFP (red fluorescence protein); eGFP (enhanced green fluorescence protein), positioned out of frame. Each target sequence contains two 20 bp regions necessary for gRNA binding and PAM motifs. Cas9n nuclease activity aided by the pair of sgRNAs leads to two individual single stranded breaks within the target sequence (indicated by an arrow), which upon non-homologous end joining repair may lead to subtle sequence deletions and thereby frame shift of the downstream GFP.

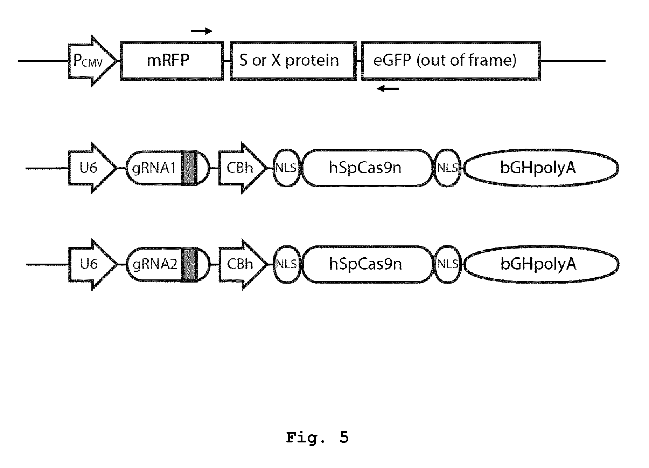

- **Figure 5**: is a schematic illustration of the reporter plasmid and two Cas9n/gRNA expression plasmids. The sgRNA expression plasmids contain a pol III promoter (U6) driving expression of the respective sgRNA, a chicken beta-actin human codon optimized promoter (CBh), Cas9 nickase (human codon optimized, D10A mutant) with two nuclear localization signals (NLS) and bovine growth hormone polyadenylation signal (bGHpolyA).

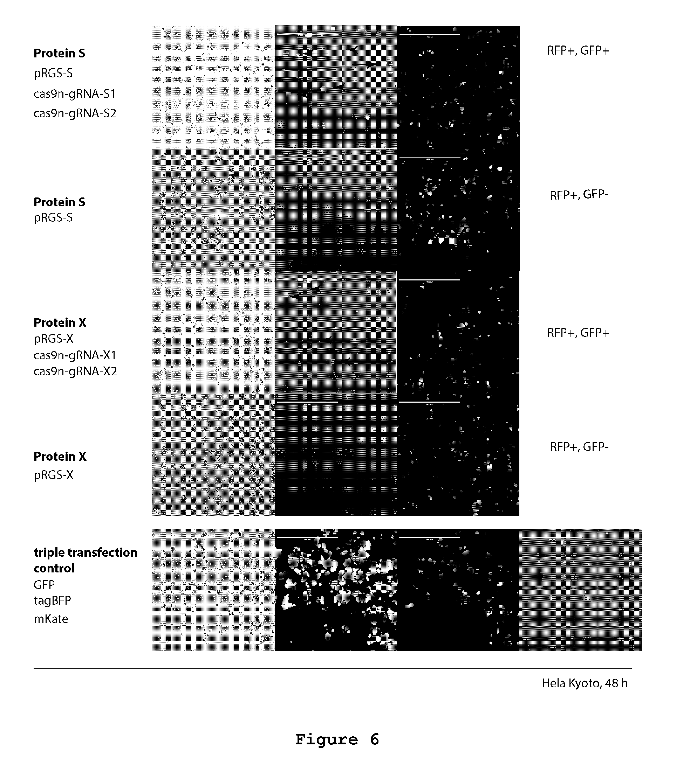

- **Figure 6**: shows Cas9n activity on HBV sequence in HeLa Kyoto cells at 48 h post transfection. Transfection with HBV reporter (pRGS-S or pRGS-X, respectively) and two expression plasmids for Cas9n/gRNAs expression; or HBV reporter plasmids alone (control); or co-transfection of individual RFP, GFP and tagBFP fluorescence reporter constructs (transfection control). Arrows indicate GFP expressing cells due to Cas9n activity. Scale bar = 400 µm.

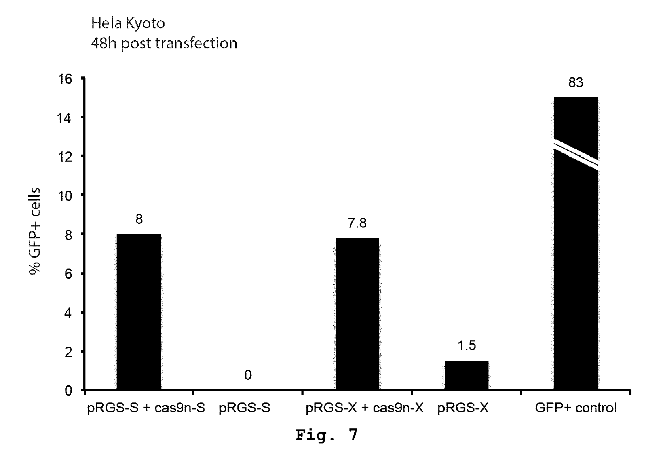

- **Figure 7**: shows quantification of the Cas9n nuclease activity in HeLa Kyoto cells at 48h post transfection. Cells containing HBV reporter for S or X protein (depicted as pRGS-S or pRGS-X) and two Cas9n/gRNA expression plasmids were analyzed by FACs. Percentage of the total GFP positive (GFP+) population is shown. Transfection control: presence of the positive control plasmid (GFP+).

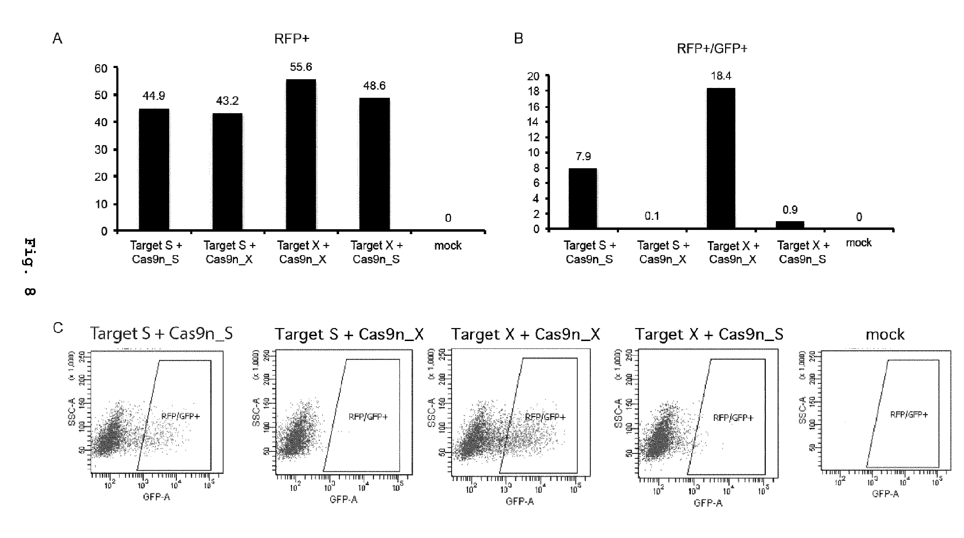

- **Figure 8**: shows detection and quantification of Cas9n activity on HBV sequence in HEK293 cells at 24h post-transfection. HEK293 cells were transfected with the respective HBV reporter (indicated as pRGS-S or pRGS-X) and two plasmids for Cas9n/gRNA expression. Negative control: Mismatched Cas9n/gRNA expression plasmids. Transfection control: GFP fluorescence reporter in an amount equal to the HBV reporter. Cells were imaged at 24h post transfection and cells expressing GFP due to Cas9n activity were detected (data not shown). (A) Cells were analyzed by FACs for RFP fluorescence to determine the presence of the HBV reporter in the transfected cells. Y-graph numbers indicate % of RFP+ cells. (B) Target specific Cas9n/gRNA nuclease activity was detected by FACs analysis of GFP+ cells in the populations of RFP+ (reporter containing) cells. (C) Graph representation of the FACs gating of the GFP+ cells among RFP+ population (quantification shown in B).

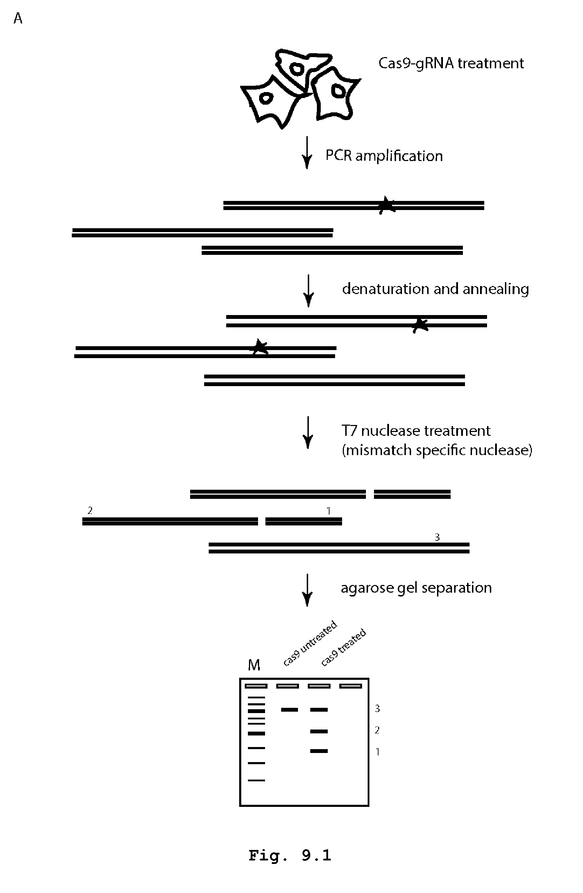

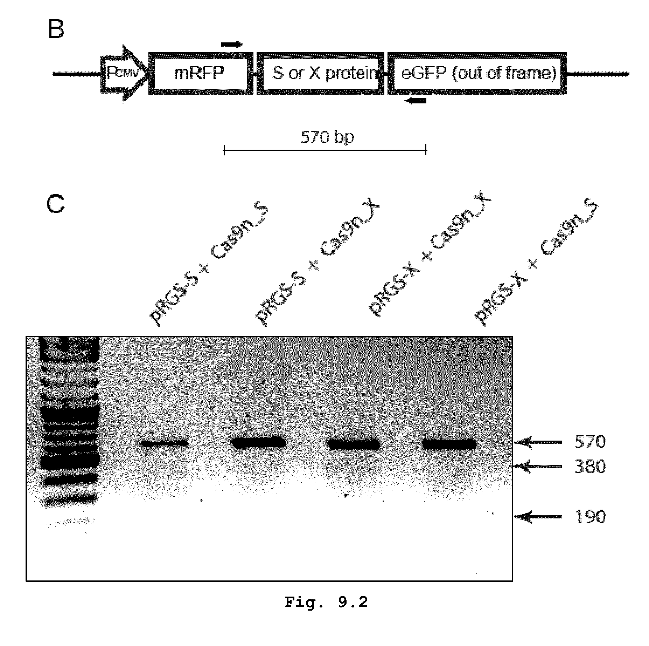

- **Figure 9**: (Figure 9.1 shows part A; Figure 9.2 shows parts B and C) demonstrates a T7 nuclease assay for detection of Cas9-mediated mutagenesis of HBV-derived target sequences. (A) T7 assay scheme to detect Cas9n/gRNA-mediated mutagenesis in mammalian cells. Target sequences encompassing the respective Cas9/gRNA cleavage site is amplified from cellular genomic DNA by PCR with locus specific primers. The PCR product is denaturated and re-annealed to form heterologous dsDNA with mismatches in the position of Cas9/gRNA-mediated mutagenesis. Mismatched positions are specifically cleaved by T7 nuclease, while wildtype PCR products remain intact. The sizes of the respective products are determined by agarose gel electrophoresis. The relative ratio of the intact versus cleaved DNA bands represent the respective proportion of the mutagenized genomic DNA and, thus, the activity and efficiency of Cas9n/gRNA cleavage of the respective target sequence. (B) T7 assay was performed using PCR primers (indicated by arrows) flanking the HBV S or X sequence in the respective reporter plasmid. (C) Assay of chromosomal DNA obtained from transfected HEK293 cells. Cells were treated with Cas9 in combination with non-specific or specific sgRNA directed towards the S or X protein sequences. Gel electrophoresis of wildtype and mutated DNA fragments after T7 assay.

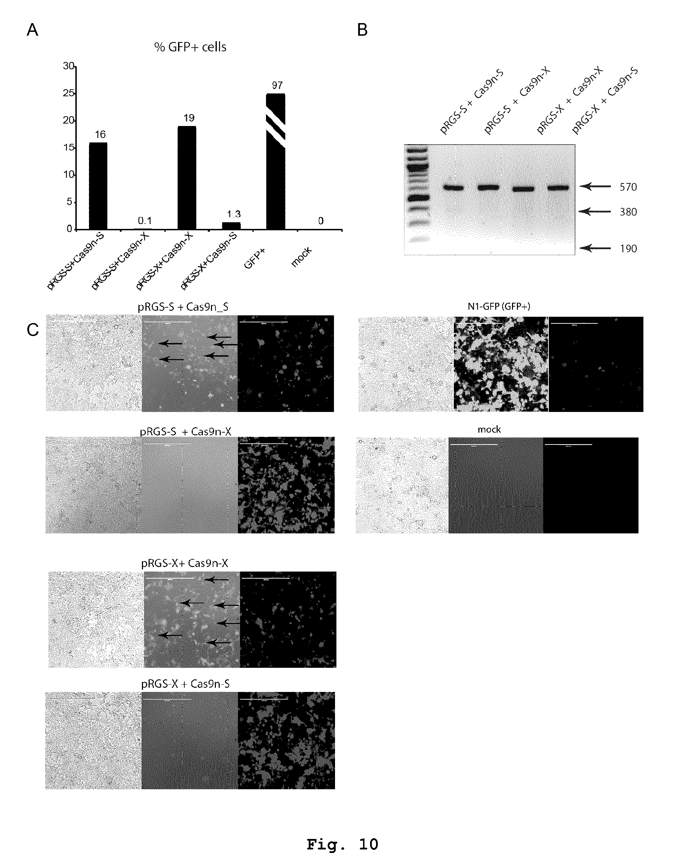

- **Figure 10**: shows Cas9n/gRNA activity in HEK293 cells at 72h post transfection. (A) FACs quantification of Cas9n-specific activity: Amount of the GFP+ cells, containing GFP frameshift caused by Cas9n-mutagenesis. Controls: quantifying GFP+ cells transfected with non-specific sgRNAs. (B) T7 detection of the Cas9n-specific activity on HBV reporter constructs. Arrows depict the sizes of wildtype and Cas9n-mutagenized DNA fragments. (C) Fluorescence microscopy visualization of the Cas9n/gRNA activity employing HBV reporter constructs. Green cells highlighted by arrows: cells containing HBV reporter with GFP frameshift mutagenesis upon Cas9n/gRNA treatment. Transfection control: constitutively expressing GFP plasmids.

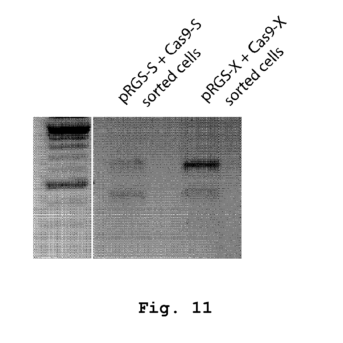

- **Figure 11**: shows a gel electrophoresis of cells that were sorted for RFP+ (reporter) and GFP+ (frameshift mutants) and subsequently used for T7 assay to detect Cas9n-specific mutagenesis.

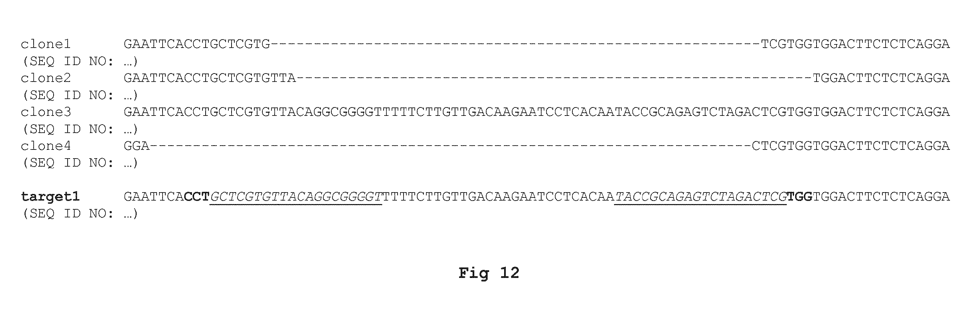

- **Figure 12**: shows Cas9n/gRNA-induced mutations (deletion) in sequences of HBV reporter DNA. The DNA was isolated from HEK 293 cells after treatment with Cas9n/gRNA and sorting for RFP (reporter) and GFP (frameshift mutation) positive cells. The bottom sequence indicates the positioning (dark grey boxes) of the respective sgRNA in the target sequence. CC and GG PAM motifs are highlighted (light grey boxes). Arrows indicate the site of the respective Cas9n/gRNA-mediated cut.

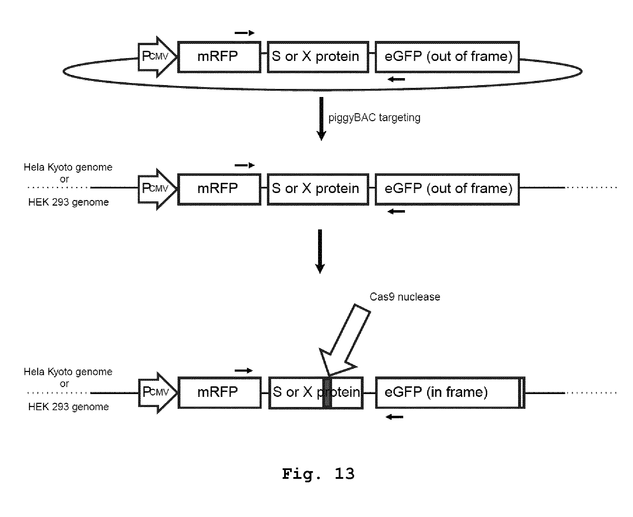

- **Figure 13**: shows schematically the generation of stable HeLa Kyoto and HEK293 cell lines containing chromosomal integrated HBV fluorescent reporter constructs. Red-Green (RFP-GFP) reporter construct containing either the sequence of the HBV S or X gene was stably integrated into the genome of HeLa Kyoto or HEK293 cells utilizing the piggyBAC (System Biosciences Inc.) targeting system. Puromycin resistant cells (resistance conveyed by the piggyBAC vector) and constitutively expressing RFP were used for assaying Cas9n/gRNA-mediated nuclease activity.

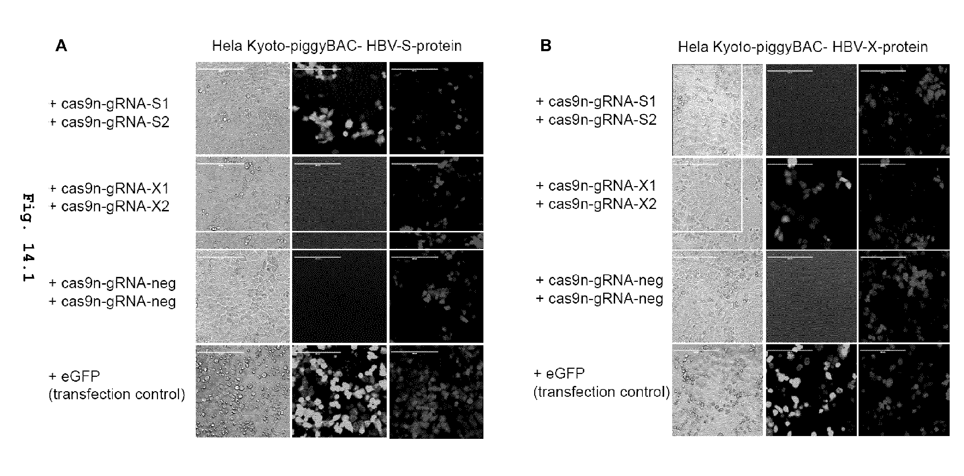

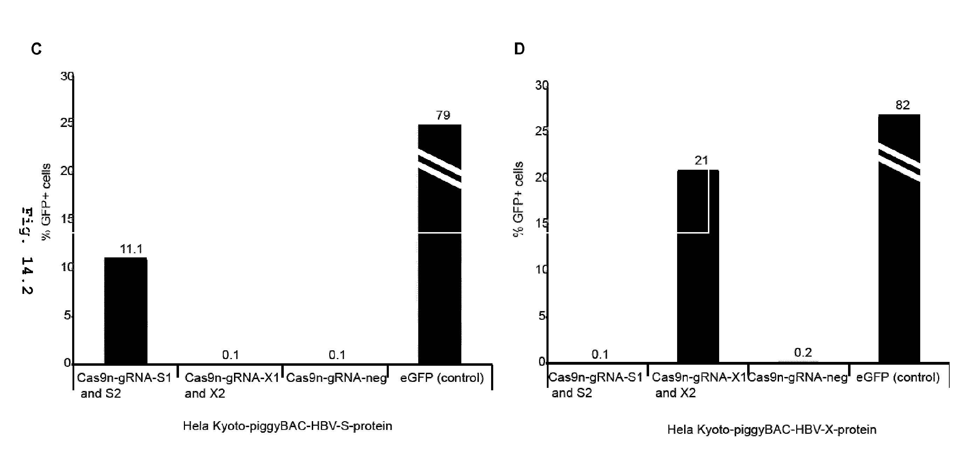

- **Figure 14**: (Figure 14.1 shows part A and B; Figure 14.2 shows parts C and D) shows Cas9n/gRNA activity in HeLa Kyoto stable cell lines containing chromosomally integrated HBV reporter constructs measured at 72h post transfection. (A) The stable HBV S protein reporter cell line cotransfected with plasmids expressing Cas9 nickase and S or X (control) protein-specific sgRNA, or negative control sgRNA (neg.). Transfection control: Transfection of a GFP-expressing vector. Fluorescence microscopy. (B) The stable HBV X protein reporter cell line transfected with Cas9n and S (control) or X sequence-specific sgRNA vectors, or negative control sgRNA (neg.) and analyzed as described before. (C) HBV S sequence-specific Cas9n/sgRNA-mediated nuclease activity was measured by FACs quantifying GFP+ cells. (D) Quantification of HBV X sequence-specific Cas9n/sgRNA-mediated nuclease activity by FACs detection of GFP+ cells.

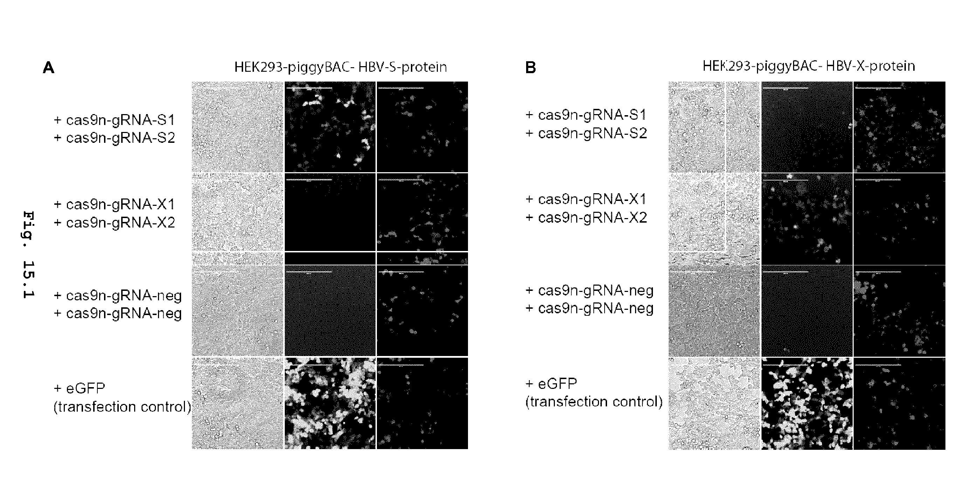

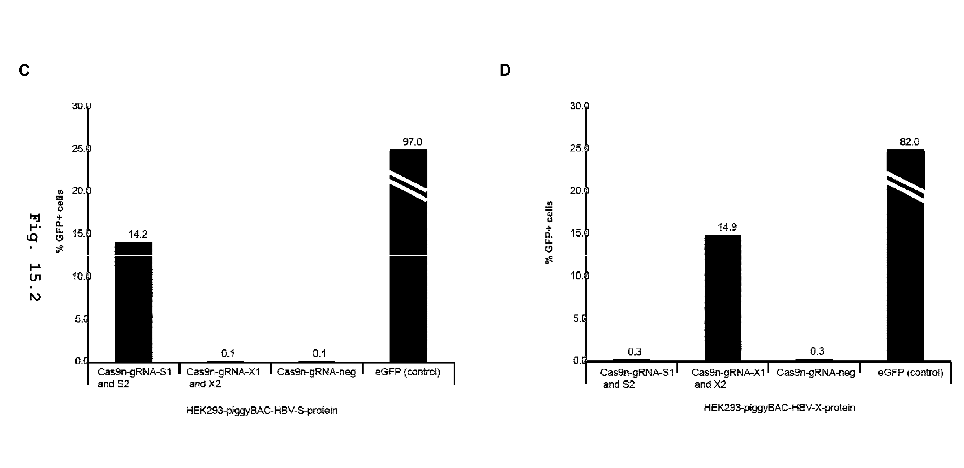

- **Figure 15**: (Figure 15.1 shows part A and B; Figure 15.2 shows parts C and D) shows Cas9n/gRNA activity in HEK293 stable cell lines containing chromosomal integrated HBV reporter constructs measured at 72h post transfection. (A) The stable HBV S protein reporter cell line co-transfected with plasmids expressing Cas9n nickase and S or X (control) protein-specific sgRNA, or negative control sgRNA (neg.). Transfection control: Transfection of a GFP-expressing vector. Fluorescence microscopy. (B). The stable HBV X protein reporter cell line was transfected with Cas9n and S (control) or X sequence-specific sgRNA vectors, or negative control sgRNA (neg.) and analyzed as described before. (C) HBV S sequence-specific Cas9n/sgRNA-mediated nuclease activity was measured by FACs quantifying GFP+ cells. (D) Quantification of HBV X sequence-specific Cas9n/sgRNA-mediated nuclease activity by FACs detection of GFP+ cells.
- **Figure 16**: shows a gel electrophoresis of cell extracts from a T7 assay for the Cas9n/sgRNA-specific activity on stable integrated HBV reporter constructs in HeLa Kyoto cell lines. Cell lines containing either the S sequence- or X sequence-specific HBV fluorescent reporter were transfected with plasmids expressing Cas9 nickase and corresponding sgRNAs. An arbitrary genomic locus and sgRNA specific to this region was used as positive control.
- **Figure 17**: shows an analysis of the conservation of the identified 23 nucleotide long (20 nt complementary to gRNA, 3 nt PAM sequence) sgRNA/Cas9 target sequences S1, S2, X1 and X2 (shown in SEQ ID NOs:13 to 16) among isolates of genotypes B, C, D, E, F and G.

### EXAMPLES

### Example 1: Identification of conserved target sites in the hepatitis B genome

A publicly available database of the HBV isolates sequences was used for the sequence conservation analysis (Hayer J. et al., 2013, Nucleic Acids Research, 41(D1):D566-D70; Hepatitis B virus database HBVdb). HBV genome was first analysed for the presence of a region with a length of at least 50 nucleotides in which nucleotide positions have high conservation values (more than 99.9%) according to the alignments dataset and frequencies repertoire across all genotypes and genotypes abundant in Europe. Analysis was performed with 1931 sequences of HBV isolates for genotype A and 3114 sequences of HBV isolates for genotype D.

Two regions in the hepatitis B virus genome that are highly conserved among HBV isolates of genotype A were selected as potential targets for HBV inactivation. The two conserved regions were analysed for the presence of potential sgRNA target sequences with immediately adjacent PAM regions (CCN-20gRNA...20gRNA-NGG), i.e. for sequences where two PAM motifs are in reverse complementary orientation and positioned at least 40 bp away from each other. Individual pairs of the gRNA+PAM were analysed for the off-target potential in human genome. Only pairs for which the closest off target sequence had at least 3 mismatches in the 20 bp region were selected.

Filtered gRNA+PAM (23 nucleotides long sequences) underwent a second round of the conservation analysis, calculating the nucleotide conservation for the total 23 nucleotide sequence for each gRNA candidate. Conservation was analysed among HBV isolates of the genotype A. Conservation was calculated by extracting the probability of the nucleotide appearance at the semi-conserved position and assigning it as a conservation value for the target sequence. If more than two semi-conserved positions occurred at the same position within the 23 nucleotide sequence, the values were calculated by multiplying two probabilities and extracting square root. gRNA+PAM sequences with the overall conservation value higher than 95% were regarded as being highly conserved.

Two conserved regions were identified within the S and X genes of the HB genome (indicated with scissors in Fig. 1, SEQ ID NOs:19 and 20, respectively). In both of these conserved sequences, a single pair of highly conserved sgRNA/Cas9n target sequences (gRNA target + PAM) was identified which fulfilled all the filtering criteria, i.e. showed more than 95% conservation over the full sequence of 23 nucleotides among 1931 isolates of the genotype A. The individual sgRNA/Cas9n target sequences are designated S1 (SEQ ID NO:13), S2 (SEQ ID NO:14), X1 (SEQ ID NO:15), and X2 (SEQ ID NO:16).

The possibility to utilize these conserved regions for inactivation of further genotypes was evaluated by calculating the conservation of the 23 nucleotide long sequences among isolates of genotypes B, C, D, E, F and G. Results are presented in the figure 17. It can be seen that the sgRNA target sequences S1, S2, X1 and X2 exhibit more than 93% conservation over the full sequence of 23 nucleotides among the analysed isolates of genotype B, C, E and more than 89% conservation over the full sequence among the analysed isolated of genotype D.

### Example 2: Molecular Cloning of reporter plasmids for detection of Cas9n nuclease activity on HBV S or X target sequences

After identification of suitable target sequences, a reporter construct for detection of Cas9n nuclease activity on these sequences was cloned. The reporter constructs make use of the following cellular mechanism: Cas9n nuclease activity aided by two sgRNAs leads to a double stranded break within the target sequence (indicated by an arrow in Figure 4), which upon non-homologous end joining repair may lead to subtle sequence deletions or insertions and thereby frame shift of a GFP gene that is located downstream from the cleavage sites. Because of codon triplets, 2 out of 3 "repairs" statistically result in "out of frame" and 1 out of 3 in "in frame" fusion of the eGFP to an RFP sequence that is located upstream of the cleavage site. Therefore, presence of the reporter is detected by the RFP signal, while nuclease activity can be detected by simultaneous RPF and GFP fluorescence.

The reporter construct, thus, contains the gene encoding RFP (red fluorescence protein) for constitutive expression of RFP, the respective conserved S or X-protein target sequence (having 73 bp or 58 bp, i.e. SEQ ID NOs:19 and 20, respectively), and the gene encoding eGFP (enhanced green fluorescence protein) which is positioned out of the reading frame in relation to the RFP sequence (Fig. 4 and 5). The S and X protein sequences contain two 20 bp regions necessary for gRNA binding and two PAM motifs each (Fig. 2 and 3).

RFP-GFP reporter was cloned according to Kim et al., 2011, Nature methods, 8(11): 941-943. The RFP-GFP reporter was based on the pEGFP-neo-GFP plasmid (Clontech). Firstly, mRFP was amplified from pDSV-mRFP (Tonelli et al., Biotechniques, 2006, 41) using the primers mRFP-F and mRFP-R (SEQ ID NOs:26-27 , respectively), and the amplified product was cloned into the *Nhe*I site of pEGFP-N1 (Clontech) functionally linked to the CMV promoter. The ORF of the eGFP was amplified omitting the ATG as start codon using the primers eGFP-F and eGFP-R (SEQ ID NOs:28-29, respectively), and was then sequentially cloned into the *Bam*HI and *Not*I site of the plasmid. Oligonucleotides that contained HBV target sites (SEQ ID NOs:47-50, respectively) were synthesized (Biomers) and annealed *in vitro.* The annealed oligonucleotides were ligated into a vector (pEGFP-N1-RFP) digested with EcoR1 and BamH1. In-frame stop codons were avoided or reduced in the HBV target sequence by changing the orientation of the target site.

### Example 3: Molecular cloning of Cas9n/sgRNA expression plasmids

Cas9n/sgRNA expression plasmids were constructed based on a pX355/crRNA/tracrRNA plasmid which contains a pol III promoter (U6) driving expression of the sgRNA, a chicken beta-actin human codon optimized promoter (CBh), Cas9 nickase (human codon optimized, D10A mutant) with two nuclear localization signals (NLS) (SEQ ID NO:23), bovine growth hormone polyadenylation signal (bGHpolyA) (Ran et. al., 2013, Cell) and a crRNA/tracrRNA part (encoding SEQ ID NO:17) (Fig. 5). sgRNA sequences comprising 20 nucleotide long gRNA sequences were cloned for each gRNA target separately (DNA encoding sgRNAs shown in SEQ ID NOs:5-8). For this purpose, complementary oligonucleotides containing a guanine nucleotide necessary for the polIII driven expression and 20 nucleotides of the corresponding gRNA (SEQ ID NOs:30-37) were synthesized (Biomers) and annealed *in vitro.* Annealed oligonucleotides were cloned into plasmid pX355 via *Bbs*I sites. Thus, the resulting four expression plasmids contained each a Cas9 nickase and one sgRNA, i.e. a hybrid of 20 bp gRNA and crRNA/tracrRNA.

### Example 4: Detection of Cas9n activity in human HeLa cells

A set of three plasmids comprising a reporter plasmid and two Cas9n/sgRNA expression plasmids was used in an assay for the detection of Cas9n activity in human cells.

The Cas9n activity on HBV sequences was shown in HeLa Kyoto cells at 48 h post transfection (Fig. 6). HeLa Kyoto cells were transfected with the HBV reporter (pRGS-S or pRGS-X, respectively) and two of the previously cloned expression plasmids for Cas9n/sgRNA expression each containing a different sgRNA (see above, i.e. one of SEQ ID NOs:13-16), together comprising a pair necessary to create a dsDNA break. The following plasmids were combined:

For evaluation of Cas9n activity on the sequence encoding protein S:

| Reporter plasmid | Cas9n/sgRNA |
|---|---|
| | expression plasmids |
| pRGS-S | cas9n-gRNA-S1 |
| (comprising SEQ ID NO:19) | (comprising SEQ ID NO:23 and 5) |
| | cas9n-gRNA-S2 |
| | (comprising SEQ ID NO:23 and 6) |

For evaluation of Cas9n activity on the sequence encoding protein X:

| Reporter plasmid | Cas9n/sgRNA |
|---|---|
| | expression plasmids |
| pRGS-X | cas9n-gRNA-X1 |
| (comprising SEQ ID NO:20) | (comprising SEQ ID NO:23 and 7) |
| | cas9n-gRNA-X2 (comprising SEQ ID NO:23 and 8) |

HBV reporter plasmids alone (i.e. omitting Cas9n/sgRNA expression plasmids) were added to the control wells. A transfection control was performed by co-transfection of individual RFP (pDSV-mRFP (Tonelli et al., Biotechniques, 2006, 41), GFP and tagBFP fluorescence reporter constructs (Evrogen). Cells were imaged 48h post transfection (Figure 6). Arrows indicate GFP expressing cells due to Cas9n activity. Scale bar = 400 µm.

### Results

Wells containing only the HBV reporter construct for protein S or protein X were RFP positive as expected, indicating the presence of the RFP-GFP reporter. The RFP gene was constitutively expressed from the HBV reporters. However, the GFP signal was not detected because of the out-of-frame position of the GFP gene in the intact HBV reporters. On the contrary, GFP signal was detected in the wells with plasmids expressing Cas9 nickase and sgRNAs along with the HBV reporter. This indicates that the Cas9 nickases guided by the pair of sgRNAs have cleaved the HBV reporter in the HBV target sequence. This, in consequence, has led to the NHEJ repair with a frame shift of the GFP relative to RFP and, in consequence, to the generation of cells expressing GFP. The control for the transfection efficiency was transfected with three independent plasmids coding for 3 fluorescence genes: RFP, GFP and tagBFP. Fluorescent microscopy revealed high transfection efficiency in all three fluorescence channels, indicating that the three plasmids are co-transfected efficiently. In consequence, co-transfection of three plasmids containing Cas9 nickase, sgRNAs and HBV reporter could be expected to be also efficient. In summary, co-transfection of the Cas9 nickase and a pair of the sgRNAs has led to the efficient cleavage of the HBV targets in the reporter in HeLa Kyoto cells.

### Example 5: Quantification of Cas9n activity in human HeLa cells

Subsequently, the Cas9n nuclease activity in HeLa Kyoto cells at 48h post transfection was quantified via FACS analysis of cells containing HBV reporter for S or X protein (pRGS-S or pRGS-X) and two Cas9n/sgRNA expression plasmids (Fig. 7). Percentage of the total GFP+ population is shown. Transfection control was measured for the presence of the positive control plasmid (GFP+).

### Results

The wells containing only HBV protein S and protein X reporter showed no or only a low amount of cells positive for GFP, i.e. 0% and 1.5% respectively (Fig.7). This result is consistent with the fluorescence imaging results, indicating that the cells containing only the reporter do not express GFP or express on a basal auto-fluorescence level. However when Cas9 and sgRNAs targeting the HBV protein S were co-transfected along with the reporter, a high fluorescence level was detected in 8% of the cells. A similar result was observed for the protein X: 7.8% of the cells expressed activated GFP reporter protein due to the Cas9 nickase treatment guided by the X-specific sgRNAs. A transfection control with constitutively GFP-expressing plasmid in an amount equal to the HBV reporter showed that the majority of the cells were efficiently transfected and 83% of the cells were GFP positive.

Importantly, only one out of three frame shifts of the GFP upon Cas9 nickase cleavage and NHEJ repair results in the GFP expression. Therefore the quantification of the GFP positive cells represents only a portion of all Cas9 cleavage events and presumably underestimates the nuclease activity. Overall, the number of GFP-positive cells was markedly increased after cotransfection of reporter plasmids and the corresponding Cas9n/sgRNA expression plasmids (Fig. 7). Consequently, the Cas9n/gRNA constructs used were able to efficiently introduce deletions leading to a frameshift in the reporter constructs.

### Example 6: Detection and Quantification of Cas9n activity in human HEK293 cells, 24 h post-transfection

Cas9n activity on the HBV sequence was, further, detected and quantified in HEK293 cells at 24h post-transfection.

HEK293 cells were transfected with the respective HBV reporter (indicated as pRGS-S or pRGS-X) and the two corresponding plasmids for Cas9n/gRNA expression essentially as described above for HeLa cells (Fig. 8). Mismatched Cas9n/gRNA expression plasmids were used in negative control experiments. Transfection control was performed by transfection of the GFP fluorescence reporter in an amount equal to the HBV reporter. Cells were imaged at 24h post transfection (data not shown). GFP positive cells were visible only in the GFP control and in the wells transfected with matching reporter and Cas9n/sgRNA plasmids (i.e. Target S protein + Cas9n_S or Target X protein + Cas9n_X).

Cells were analyzed by FACs for RFP fluorescence to determine the presence of the HBV reporter in the transfected cells (Fig. 8 A). Furthermore, the target specific Cas9n/gRNA nuclease activity was detected by FACs analysis of GFP+ cells in the populations of RFP+ (reporter containing) cells. A quantification of the FACs gating of the GFP+ cells among the RFP+ population is shown in Figure 8B.

### Results

GFP expression was successfully induced in cells transfected with the respective HBV reporter (indicated as pRGS-S or pRGS-X) and the two corresponding plasmids for Cas9n/sgRNA expression, while no GFP expression was observed in cells containing only reporter plasmid or mismatched pairs of reporter and Cas9n/sgRNA expression plasmids (e.g. pRGS-S and Cas9n-sgRNA-X1/X2).

In order to normalize to the transfection efficiency and calculate the amount of the GFP cells among cells that received the HBV reporter, cells were first sorted for the constitutively expressed reporter RFP. While the number of RFP positive cells was substantially the same in cells transfected with matching and mismatching pairs of reporter and Cas9n/gRNA expression plamids (Fig. 8 A), the number of RFP and GFP positive cells was substantially increased in cells transfected with matching pairs of reporter and Cas9n/gRNA expression plamids (Fig. 8B and C). This indicated that the Cas9 nickase guided by specific sgRNAs produced efficient deletions in HBV reporter constructs in HEK cells.

### Example 7: Detection of Cas9n activity in human HEK293 cells with a T7 nuclease assay

A T7 nuclease assay was carried out for the detection of Cas9 nickase-mediated mutagenesis of the HBV-derived target sequences in HBV reporter constructs (Figure 9A). For that, total DNA (chromosomal + episomal) was obtained from HEK293 cells that were transfected with HBV reporter constructs and treated with Cas9 nickase in combination with sgRNA that was not specific or specific for the S or X protein sequences. The PCR primers T7-RFP and T7-GFP (SEQ ID NOs:38-39, respectively) flanked the HBV S or X sequence in the respective reporter plasmid (indicated by arrows in Fig. 9B). The PCR product was denaturated and re-annealed to form heterologous dsDNA with mismatches in the position of Cas9/gRNA-mediated mutagenesis. Mismatched positions were then specifically cleaved by T7 nuclease, while wildtype PCR products remained intact. The sizes of the respective products were subsequently determined by agarose gel electrophoresis (Figure 9C). The relative ratio of the intact versus cleaved DNA bands represents the respective proportion of the mutagenized genomic DNA and, thus, the activity and efficiency of Cas9n/gRNA cleavage of the respective target sequence.

### Supplementary Experiment

The T7 assay as outlined above was additionally carried out with HEK293 cells that were sorted for GFP positive cells via FACS prior to the assay. After sorting, the cells that were RFP+ (reporter) and GFP+ (frameshift mutants) were used for the T7 assay to detect Cas9n-specific mutagenesis.

### Results

As can be seen from Figure 9C, smaller bands, representing the nuclease-cleaved DNA strands into which a DNA mismatch was introduced by the Cas9n/gRNA system, were only present in cell samples that were transfected with a matching pair of reporter plasmid and Cas9n/gRNA expression plasmid (i.e. pRGS-S + Cas9n_S and pRGS-X + Cas9n_X).

The results of the experiments in HEK293 cells sorted for GFP are shown in Figure 11. Here, it can be seen that smaller bands, representing the nuclease-cleaved DNA strands into which a DNA mismatch was introduced by the Cas9n/gRNA system, were present to a considerable extend in cell samples that were transfected with a matching pair of reporter plasmid and Cas9n/gRNA expression plasmid (i.e. pRGS-S + Cas9n_S and pRGS-X + Cas9n_X).

In summary, it was demonstrated that the tested sgRNAs specifically targeted the HBV protein to which they were complementary, i.e. sgRNAs S1 and S2 (SEQ ID NOs:1 and 2, respectively) introduced DNA mismatches into the gene coding for the S protein and sgRNAs X1 and X2 (SEQ ID NOs:3 and 4, respectively) introduced DNA mismatches into the gene coding for the X protein.

### Example 8: Detection and Quantification of Cas9n activity in human HEK293 cells, 72 h post-transfection

Cas9n/gRNA activity was also determined in HEK293 cells 72h post transfection. HEK293 cells were transfected as described above. FACS analysis and T7 nuclease assay were carried out essentially as described above.

### Results

FACs quantification of Cas9n-specific activity showed that GFP expression was successfully induced in cells transfected with the respective HBV reporter (indicated as pRGS-S or pRGS-X) and the two corresponding plasmids for Cas9n/gRNA expression (Fig. 10A and C), while no GFP expression was observed in cells containing mismatched pairs of reporter and Cas9n/gRNA expression plamids (e.g. pRGS-S and cas9n-gRNA-X1/X2). Controls were performed by quantifying GFP+ cells in wells transfected with non-specific sgRNAs for the FACS analysis. Constitutively expressing GFP plasmids were used as transfection control for the fluorescence microscopy.

Moreover, T7 nuclease assay detection of the Cas9n-specific activity on HBV reporter constructs showed wildtype and Cas9n-mutagenized DNA fragments (Fig. 10B, arrows, 570 bp: wildtype; 380 and 190 bp: Cas9n-mutagenized DNA fragments).

### Example 9: Sequence analysis of human HEK293 cells, 72 h post-transfection with Cas9n/sgRNA and reporter plasmid

Asequence analysis of transfected HEK293 was carried out to determine the deletions that were induced in the genome.

HEK 293 cells containing HBV reporter were treated with Cas9n/sgRNA and sorted for RFP (reporter) and GFP (frameshift mutation) positive cells. HBV reporter DNA was isolated and subsequently sequenced. The sequences of four clones are shown in Fig. 12 (SEQ ID NOs:40-43). For comparison, the corresponding sequence in the HBV S gene is also shown in Fig.12 (SEQ ID NO:44).

### Results

The four clones shown demonstrate the presence of Cas9n/sgRNA-induced mutations (i.e. deletions) in sorted GFP+ cells (Fig. 12). Deletions were introduced into 3 out of four clones, i.e. clone 1, 2, and 4. The deletions have different lengths due to the error prone cellular NHEJ repair mechanism. A long span of the deletion coincides with the mechanism of the Cas9 nickase cleavage. Cas nickase cleaves the target sequence in one strand 3 bp upstream of the PAM region (Fig. 12, PAM indicated in bold letters). The gRNAs were designed to anneal to opposite strands and have an offset of 27 nucleotides in target S. Therefore, the cleavage also happens on strands of the DNA opposite to each other, thus creating a single stranded 5'overhang in the protein S target. The 5'overhang is prone to deletion via NHEJ and therefore in observed mutated clones the deletions constitute almost the perfect sequence of the 5' overhang.

### Example 10: Generation of stable HeLa Kyoto and HEK293 cell lines containing chromosomal integrated HBV fluorescent reporter constructs.

### Stable Hela and HEK cell lines

A red-Green (RFP-GFP) reporter construct containing either the sequence of the HBV S or X gene was stably integrated into the genome of HeLa Kyoto or HEK293 cells using the piggy-BAC (System Biosciences Inc.) targeting system (Fig. 13). For that, a portion of the RFP-GFP reporter containing fluorescence and target sequences were subcloned into piggyBAC vector via NheI and BstBI, generating piggy-BAC-HBV-S or piggy-BAC-HBV-X. The piggyBAC vector comprises a puromycin resistance gene.

Stable Hela and HEK cell lines were generated by transfecting the piggy-BAC-HBV-S or piggy-BAC-HBV-X and selecting for puromycin resistance (2 µg/ml) for 10 days. Cells were controlled for the RFP expression. Puromycin resistant cells which were constitutively expressing RFP were subsequently used for assaying Cas9n/sgRNA-mediated nuclease activity.

### HeLa cell lines

The stable HBV S protein reporter HeLa cell line was cotransfected with plasmids expressing Cas9 nickase and S or X (control) protein-specific sgRNA, or negative control sgRNA (neg.) (Fig. 14A). Transfection of a GFP-expressing vector served as transfection control experiment. Transfected cultures were analyzed by fluorescence microscopy. Likewise, the stable HBV X protein reporter cell line was transfected with Cas9n and S (control) or X sequence-specific sgRNA vectors, or negative control sgRNA (neg.) and analyzed as described before (Fig. 14B).

HBV S sequence-specific Cas9n/sgRNA-mediated nuclease activity was measured by FACs quantifying GFP+ cells (Fig. 14C). HBV X sequence-specific Cas9n/gRNA-mediated nuclease activity was quantified by FACs detection of GFP+ cells (Fig. 14D).

### HEK293 cell lines

Similarly, the stable HBV S protein reporter HEK293 cell line was cotransfected with plasmids expressing Cas9 nickase (Cas9n) and S or X (control) protein-specific sgRNA, or negative control sgRNA (neg.) (Fig. 15). Transfection of a GFP-expressing vector served as transfection control experiment. Transfected cultures were analyzed by fluorescence microscopy (Fig. 15A). Likewise, the stable HBV X protein reporter HEK293 cell line was transfected with Cas9n and S (control) or X sequence-specific sgRNA vectors, or negative control sgRNA (neg.) and analyzed as described before (Fig. 15B).

HBV S sequence-specific Cas9n/gRNA-mediated nuclease activity was subsequently measured by FACs quantifying GFP+ cells (Fig. 15C). Further, HBV X sequence-specific Cas9n/gRNA-mediated nuclease activity was quantified by FACs detection of GFP+ cells.

### Results HeLa cell lines

The Cas9n/gRNA activity in stable HeLa Kyoto cell lines containing chromosomally integrated HBV reporter constructs at 72h post transfection is shown in Fig. 14. It can be seen that the number of GFP expressing cells is considerably increased in samples that comprised the two plasmids for Cas9n/gRNA expression that correspond to the reporter protein that is stably expressed in the cells (e.g. cas9n-gRNA-S1 and cas9n-gRNA-S2 in HeLa Kyoto cell line that stably expresses S protein).

### Results HEK293 cell lines

The Cas9n/gRNA activity in stable HEK293 cell lines containing chromosomally integrated HBV reporter constructs at 72h post transfection is shown in Fig. 15. It can be seen that the number of GFP expressing cells is considerably increased in samples that comprised the two plasmids for Cas9n/gRNA expression that correspond to the reporter protein that is stably expressed in the cells (e.g. cas9n-gRNA-S1 and cas9n-gRNA-S2 in HeLa Kyoto cell line that stably expresses S protein).

### Example 11: Detection of Cas9n activity in HeLa Kyoto cell lines containing stable integrated HBV reporter constructs with a T7 nuclease assay

The T7 nuclease assay for the Cas9n/gRNA-specific activity was carried out on the HeLa Kyoto cells containing stable integrated HBV reporter constructs as described above.

Cell lines containing either the S sequence- or X sequence-specific HBV fluorescent reporter were transfected with plasmids expressing Cas9 nickase and corresponding sgRNAs. An arbitrary genomic locus, that was previously tested for efficient Cas9n targeting, and sgRNA specific to this region was used as positive control (sgRNA-control).

### Results

As can be seen from Fig. 16 smaller bands, representing the nuclease-cleaved DNA strands into which a DNA mismatch was introduced by the Cas9/gRNA system, were only present in cell samples that were transfected with a pair of Cas9/gRNA expression plamids that matches the stably expressed protein (e.g. gRNA-S1 and gRNA-S2 in HeLa Kyoto cell line that stably expresses S protein). Thus, it was demonstrated that the tested sgRNAs specifically targeted the HBV protein to which they were complementary, i.e. sgRNAs S1 and S2 (SEQ ID NOs:1 and 2, respectively) introduced DNA mismatches into the gene coding for the S protein and sgRNAs X1 and X2 (SEQ ID NOs:3 and 4, respectively) introduced DNA mismatches into the gene coding for the X protein.

## Claims

1. DNA-binding molecule which is able to specifically bind to a predetermined nucleotide sequence within the hepatitis B virus genome for use in a method of treating and/or preventing a hepatitis B and/or a hepatitis D virus infection in a patient, wherein said method comprises the administration of the DNA-binding molecule and a nuclease, wherein the nuclease is guided by the DNA-binding molecule to said predetermined nucleotide sequence where it cleaves the DNA to disrupt the hepatitis B virus genome.

2. DNA-binding molecule for use in a method according to claim 1, wherein said DNA-binding molecule comprises
a) a DNA-binding nucleic acid sequence, such as a DNA-binding nucleic acid sequence which comprises a CRISPR RNA (crRNA); or
b) a DNA-binding polypeptide sequence, such as a polypeptide sequence derived from a zinc finger polypeptide or a transcription activator-like effector polypeptide.

3. DNA-binding molecule for use in a method of claims 1 to 2, wherein the nuclease is selected from the group consisting of Type II restriction endonucleases, Type IIS endonucleases, such as the FokI cleavage domain, Cas9, Cas9-derived nickase (Cas9n) and functional fragments or variants thereof.

4. DNA binding molecule for use in a method of claims 1 to 3, wherein two DNA binding molecules are administered in combination with a nuclease that introduces single strand breaks, such as a Cas9 derived nickase (Cas9n).

5. DNA-binding molecule for use in a method of claims 1 to 4, wherein the predetermined sequence of the hepatitis B virus genome is a sequence which is conserved within a hepatitis B virus genotype and, preferably, conserved between different hepatitis B virus genotypes.

6. DNA-binding molecule for use in a method of claims 1 to 5, wherein the predetermined nucleotide sequence within the hepatitis B virus genome comprises or consists of 17 to 20 consecutive nucleotides of positions 1-20 of any of SEQ ID NO: 13, 14, 15, or 16, or variants or complements thereof.

7. DNA-binding molecule for use in a method of claims 1 to 6, which is an oligoribonucleotide, wherein the oligoribonucleotide preferably comprises less than 200 nucleotides.

8. DNA-binding molecule for use in a method of claim 7, wherein the DNA-binding sequence of the DNA-binding molecule has a length of 16 to 20 nucleotides.

9. DNA-binding molecule for use in a method of claims 7 to 8, wherein said DNA-binding sequence of the DNA-binding molecule comprises or consists of a sequence set forth in SEQ ID NOs :9-12 or variants or complements thereof.

10. DNA-binding molecule for use in a method of claims 7 to 9, wherein said DNA-binding molecule comprises a crRNA sequence, preferably a crRNA/tracrRNA hybrid sequence, such as SEQ ID NO: 17.

11. DNA-binding molecule for use in a method of claims 9 to 13, wherein said DNA-binding molecule comprises or consists of a sequence set forth in SEQ ID NO: 1-4 or variants or complements thereof.

12. Oligoribonucleotide which comprises a sequence set forth in SEQ ID NOs:9-12 or variants or complements thereof, wherein the oligonucleotide preferably further comprises a crRNA sequence, and more preferably a crRNA/tracrRNA hybrid sequence.

13. Vector encoding a DNA-binding molecule of claims 1 to 11 or an oligoribonucleotide of claim 12, optionally further encoding a nuclease.

14. Pharmaceutical composition comprising the DNA-binding molecule of claims 1-11, the oligoribonucleotide of claim12 and/or the vector of claim 13.

15. Kit comprising
a1) at least one DNA-binding molecule which is able to specifically bind to a predetermined nucleotide sequence within the hepatitis B virus genome, such as the DNA-binding molecule of any of claims 1-11; or
a2) at least one oligoribonucleotide comprising a sequence set forth in SEQ ID NOs:9-12 or variants or complements thereof, such as the oligoribonucleotide according claim 12; and
b) at least one nuclease which is guided by the DNA-binding molecule to said predetermined nucleotide sequence where it cleaves the DNA to disrupt the hepatitis B virus genome.

16. *In vitro* method for the disruption of a hepatitis B virus genome and/or a hepatitis D virus genome, comprising contacting the genome with
(a) at least one DNA-binding molecule which is able to specifically bind to a predetermined nucleotide sequence within the hepatitis B virus genome, and
(b) at least one nuclease which is guided by the DNA-binding molecule to said predetermined nucleotide sequence where it cleaves the DNA to disrupt the hepatitis B virus genome.

17. Use of a kit of claim 15 or a DNA-binding molecule which is able to specifically bind to a predetermined nucleotide sequence within the hepatitis B virus genome for the *in vitro* inactivation of a hepatitis B virus genome and/or a hepatitis D virus genome, wherein a nuclease is guided by the DNA-binding molecule to said predetermined nucleotide sequence where it cleaves the DNA to disrupt the hepatitis B virus genome, wherein said DNA-binding molecule preferably comprises of a sequence set forth in SEQ ID NOs:9-12 or variants or complements thereof.


SEQUENCE LISTING

<110>  Heinrich-Pette-Institut - Leibnitz-Insitut für
       Experimentelle Virologie

<120>  HBV Inactivation

<130>  P 94147

<160>  50

<170>  PatentIn version 3.5

<210>  1
<211>  103
<212>  RNA
<213>  artificial

<220>
<223>  sgRNA with S1 gRNA

<400>  1
gaccccgccu guaacacgag cguuuuagag cuagaaauag caaguuaaaa uaaggcuagu      60

ccguuaucaa cuugaaaaag uggcaccgag ucggugcuuu uuu                       103


<210>  2
<211>  103
<212>  RNA
<213>  artificial

<220>
<223>  sgRNA with S2 gRNA

<400>  2
guaccgcaga gucuagacuc gguuuuagag cuagaaauag caaguuaaaa uaaggcuagu      60

ccguuaucaa cuugaaaaag uggcaccgag ucggugcuuu uuu                       103


<210>  3
<211>  103
<212>  RNA
<213>  artificial

<220>
<223>  sgRNA with X1 gRNA

<400>  3
ggucugugcc uucucaucug cguuuuagag cuagaaauag caaguuaaaa uaaggcuagu      60

ccguuaucaa cuugaaaaag uggcaccgag ucggugcuuu uuu                       103


<210>  4
<211>  103
<212>  RNA
<213>  artificial

<220>
<223>  sgRNA with X2 gRNA

<400>  4
gguaaagaga ggugcgcccc gguuuuagag cuagaaauag caaguuaaaa uaaggcuagu      60

ccguuaucaa cuugaaaaag uggcaccgag ucggugcuuu uuu                        103


<210>  5
<211>  103
<212>  DNA
<213>  artificial

<220>
<223>  DNA sequence encoding sgRNA with S1 gRNA

<400>  5
gaccccgcct gtaacacgag cgttttagag ctagaaatag caagttaaaa taaggctagt      60

ccgttatcaa cttgaaaaag tggcaccgag tcggtgcttt ttt                        103


<210>  6
<211>  103
<212>  DNA
<213>  artificial

<220>
<223>  DNA sequence encoding sgRNA with S2 gRNA

<400>  6
gtaccgcaga gtctagactc ggttttagag ctagaaatag caagttaaaa taaggctagt      60

ccgttatcaa cttgaaaaag tggcaccgag tcggtgcttt ttt                        103


<210>  7
<211>  103
<212>  DNA
<213>  artificial

<220>
<223>  DNA sequence encoding sgRNA with X1 gRNA

<400>  7
ggtctgtgcc ttctcatctg cgttttagag ctagaaatag caagttaaaa taaggctagt      60

ccgttatcaa cttgaaaaag tggcaccgag tcggtgcttt ttt                        103


<210>  8
<211>  103
<212>  DNA
<213>  artificial

<220>
<223>  DNA sequence encoding sgRNA with X2 gRNA

<400>  8
ggtaaagaga ggtgcgcccc ggttttagag ctagaaatag caagttaaaa taaggctagt      60

ccgttatcaa cttgaaaaag tggcaccgag tcggtgcttt ttt                        103


<210>  9

<211>  20
<212>  RNA
<213>  artificial

<220>
<223>  gRNA-S1: 1-20 gRNA

<400>  9
accccgccug uaacacgagc                                                    20


<210>  10
<211>  20
<212>  RNA
<213>  artificial

<220>
<223>  gRNA-S2: 1-20 gRNA

<400>  10
uaccgcagag ucuagacucg                                                    20


<210>  11
<211>  20
<212>  RNA
<213>  artificial

<220>
<223>  gRNA-X1: 1-20 gRNA

<400>  11
gucugugccu ucucaucugc                                                    20


<210>  12
<211>  20
<212>  RNA
<213>  artificial

<220>
<223>  gRNA-X2: 1-20 gRNA

<400>  12
guaaagagag gugcgccccg                                                    20


<210>  13
<211>  23
<212>  DNA
<213>  artificial

<220>
<223>  gRNA-S1: 1-20 gRNA, 21-23 PAM

<400>  13
accccgcctg taacacgagc agg                                                23


<210>  14
<211>  23
<212>  DNA
<213>  artificial

<220>
<223>  gRNA-S2: 1-20 gRNA, 21-23 PAM

<400>  14
taccgcagag tctagactcg tgg                                              23


<210>  15
<211>  23
<212>  DNA
<213>  artificial

<220>
<223>  gRNA-X1: 1-20 gRNA, 21-23 PAM

<400>  15
gtctgtgcct tctcatctgc cgg                                              23


<210>  16
<211>  23
<212>  DNA
<213>  artificial

<220>
<223>  gRNA-X2: 1-20 gRNA, 21-23 PAM

<400>  16
gtaaagagag gtgcgccccg tgg                                              23


<210>  17
<211>  82
<212>  RNA
<213>  artificial

<220>
<223>  crRNA/tracrRNA part in sgRNA

<400>  17
guuuuagagc uagaaauagc aaguuaaaau aaggcuaguc cguuaucaac uugaaaaagu      60

ggcaccgagu cggugcuuuu uu                                               82


<210>  18
<211>  100
<212>  RNA
<213>  artificial

<220>
<223>  hybrid gRNA crRNA/tracrRNA


<220>
<221>  N_region
<222>  (2)..(18)
<223>  gRNA region (position 2 to 18); n can be a or g or c or t/u,
      unknown, or other; gRNA region can be from 17 to 20 nucleotides

<220>
<221>  misc_feature

<222>  (2)..(18)
<223>  n is a, c, g, or u

<400>  18
gnnnnnnnnn nnnnnnnngu uuuagagcua gaaauagcaa guuaaaauaa ggcuaguccg      60

uuaucaacuu gaaaaagugg caccgagucg gugcuuuuuu                            100


<210>  19
<211>  73
<212>  DNA
<213>  Hepatitis B virus

<400>  19
cctgctcgtg ttacaggcgg ggtttttctt gttgacaaga atcctcacaa taccgcagag      60

tctagactcg tgg                                                          73


<210>  20
<211>  57
<212>  DNA
<213>  Hepatitis B virus

<400>  20
ccacggggcg cacctctctt tacgcggact ccccgtctgt gccttctcat ctgccgg         57


<210>  21
<211>  3221
<212>  DNA
<213>  Hepatitis B virus

<400>  21
gaattccact gccttccacc aaactctgca ggatcccaga gtcaggggtc tgtatcttcc      60

tgctggtggc tccagttcag gaacagtaaa ccctgctccg aatattgcct ctcacatctc     120

gtcaatctcc gcgaggactg gggaccctgt gacgaacatg gagaacatca catcaggatt     180

cctaggaccc ctgctcgtgt tacaggcggg gtttttcttg ttgacaagaa tcctcacaat     240

accgcagagt ctagactcgt ggtggacttc tctcaatttt ctagggggat ctcccgtgtg     300

tcttggccaa aattcgcagt ccccaacctc caatcactca ccaacctcct gtcctccaat     360

ttgtcctggt tatcgctgga tgtgtctgcg gcgttttatc atattcctct tcatcctgct     420

gctatgcctc atcttcttat tggttcttct ggattatcaa ggtatgttgc ccgtttgtcc     480

tctaattcca ggatcaacaa caaccagtac gggaccatgc aaaacctgca cgactcctgc     540

tcaaggcaac tctatgtttc cctcatgttg ctgtacaaaa cctacggatg gaaattgcac     600

ctgtattccc atcccatcgt cctgggcttt cgcaaaatac ctatgggagt gggcctcagt     660

ccgtttctct tggctcagtt tactagtgcc atttgttcag tggttcgtag ggctttcccc     720

cactgtttgg ctttcagcta tatggatgat gtggtattgg gggccaagtc tgtacagcat     780

cgtgagtccc tttataccgc tgttaccaat tttcttttgt ctctgggtat acatttaaac     840

cctaacaaaa caaaaagatg gggttattcc ctaaacttca tgggctacat aattggaagt 900
tggggaactt tgccacagga tcatattgta caaaagatca aacactgttt tagaaaactt 960
cctgttaaca ggcctattga ttggaaagta tgtcaaagaa ttgtgggtct tttgggcttt 1020
gctgctccat ttacacaatg tggatatcct gccttaatgc ctttgtatgc atgtatacaa 1080
gctaaacagg ctttcacttt ctcgccaact tacaaggcct ttctaagtaa acagtacatg 1140
aacctttacc ccgttgctcg gcaacggcct ggtctgtgcc aagtgtttgc tgacgcaacc 1200
cccactggct ggggcttggc cataggccat cagcgcatgc gtggaacctt tgtggctcct 1260
ctgccgatcc atactgcgga actcctagcc gcttgttttg ctcgcagccg gtctggagca 1320
aagctcatcg gaactgacaa ttctgtcgtc ctctcgcgga aatatacatc gtttccatgg 1380
ctgctaggct gtactgccaa ctggatcctt cgcgggacgt cctttgttta cgtcccgtcg 1440
gcgctgaatc ccgcggacga cccctctcgg ggccgcttgg gactctctcg tccccttctc 1500
cgtctgccgt tccagccgac cacggggcgc acctctcttt acgcggtctc cccgtctgtg 1560
ccttctcatc tgccggtccg tgtgcacttc gcttcacctc tgcacgttgc atggagacca 1620
ccgtgaacgc ccatcagatc ctgcccaagg tcttacataa gaggactctt ggactcccag 1680
caatgtcaac gaccgacctt gaggcctact tcaaagactg tgtgtttaag gactgggagg 1740
agctggggga ggagattagg ttaaaggtct ttgtattagg aggctgtagg cacaaattgg 1800
tctgcgcacc agcaccatgc aactttttca cctctgccta atcatctctt gtacatgtcc 1860
cactgttcaa gcctccaagc tgtgccttgg gtggctttgg ggcatggaca ttgaccctta 1920
taaagaattt ggagctactg tggagttact ctcgtttttg ccttctgact tctttccttc 1980
cgtcagagat ctcctagaca ccgcctcagc tctgtatcga gaagccttag agtctcctga 2040
gcattgctca cctcaccata ctgcactcag gcaagccatt ctctgctggg gggaattgat 2100
gactctagct acctgggtgg gtaataattt ggaagatcca gcatctaggg atcttgtagt 2160
aaattatgtt aatactaacg tgggtttaaa gatcaggcaa ctattgtggt ttcatatatc 2220
ttgccttact tttggaagag agactgtact tgaatatttg gtctctttcg gagtgtggat 2280
tcgcactcct ccagcctata gaccaccaaa tgcccctatc ttatcaacac ttccggaaac 2340
tactgttgtt agacgacggg accgaggcag gtcccctaga agaagaactc cctcgcctcg 2400
cagacgcaga tctccatcgc cgcgtcgcag aagatctcaa tctcgggaat ctcaatgtta 2460
gtattccttg gactcataag gtgggaaact ttacggggct ttattcctct acagtaccta 2520
tctttaatcc tgaatggcaa actccttcct ttcctaagat tcatttacaa gaggacatta 2580
ttaataggtg tcaacaattt gtgggccctc tcactgtaaa tgaaaagaga agattgaaat 2640
taattatgcc tgctagattc tatcctaccc acactaaata tttgccctta gacaaaggaa 2700
ttaaacctta ttatccagat caggtagtta atcattactt ccaaaccaga cattatttac 2760

atactctttg gaaggctggt attctatata agcgggaaac cacacgtagc gcatcatttt     2820

gcgggtcacc atattcttgg gaacaagagc tacagcatgg gaggttggtc atcaaaacct     2880

cgcaaaggca tggggacgaa tctttctgtt cccaatcctc tgggattctt tcccgatcat     2940

cagttggacc ctgcattcgg agccaactca aacaatccag attgggactt caaccccgtc     3000

aaggacgact ggccagcagc caaccaagta ggagtgggag cattcgggcc aaggctcacc     3060

cctccacacg gcggtatttt ggggtggagc cctcaggctc agggcatatt gaccacagtg     3120

tcaacaattc ctcctcctgc ctccaccaat cggcagtcag gaaggcagcc tactcccatc     3180

tctccacctc taagagacag tcatcctcag gccatgcagt g                         3221

<210>  22
<211>  1368
<212>  PRT
<213>  artificial

<220>
<223>  Cas9 nickase D10A protein, modified from Streptococcus pyogenes
       Cas9n protein

<400>  22

Met Asp Lys Lys Tyr Ser Ile Gly Leu Ala Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg

385                 390                 395                 400

Lys Gln Arg Thr Phe Asp Asn Gly Ser Ile Pro His Gln Ile His Leu
            405                 410                 415

Gly Glu Leu His Ala Ile Leu Arg Arg Gln Glu Asp Phe Tyr Pro Phe
        420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
            485                 490                 495

Asn Phe Asp Lys Asn Leu Pro Asn Glu Lys Val Leu Pro Lys His Ser
        500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
            565                 570                 575

Ser Val Glu Ile Ser Gly Val Glu Asp Arg Phe Asn Ala Ser Leu Gly
        580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys Tyr Pro Lys Leu Glu Ser Glu Phe
        995                 1000                1005

Val Tyr Gly Asp Tyr Lys Val Tyr Asp Val Arg Lys Met Ile Ala
    1010                1015                1020

Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

<210>  23

<211>  4107
<212>  DNA
<213>  artificial

<220>
<223>  Cas9 nickase D10A, modified from Streptococcus pyogenes Cas9
       protein

<400>  23
atggacaaga agtacagcat cggcctggcc atcggcacca actctgtggg ctgggccgtg      60

atcaccgacg agtacaaggt gcccagcaag aaattcaagg tgctgggcaa caccgaccgg     120

cacagcatca agaagaacct gatcggagcc ctgctgttcg acagcggcga aacagccgag     180

gccacccggc tgaagagaac cgccagaaga agatacacca gacggaagaa ccggatctgc     240

tatctgcaag agatcttcag caacgagatg gccaaggtgg acgacagctt cttccacaga     300

ctggaagagt ccttcctggt ggaagaggat aagaagcacg agcggcaccc catcttcggc     360

aacatcgtgg acgaggtggc ctaccacgag aagtacccca ccatctacca cctgagaaag     420

aaactggtgg acagcaccga caaggccgac ctgcggctga tctatctggc cctggcccac     480

atgatcaagt tccggggcca cttcctgatc gagggcgacc tgaaccccga caacagcgac     540

gtggacaagc tgttcatcca gctggtgcag acctacaacc agctgttcga ggaaaacccc     600

atcaacgcca gcggcgtgga cgccaaggcc atcctgtctg ccagactgag caagagcaga     660

cggctggaaa atctgatcgc ccagctgccc ggcgagaaga agaatggcct gttcggcaac     720

ctgattgccc tgagcctggg cctgaccccc aacttcaaga gcaacttcga cctggccgag     780

gatgccaaac tgcagctgag caaggacacc tacgacgacg acctggacaa cctgctggcc     840

cagatcggcg accagtacgc cgacctgttt ctggccgcca agaacctgtc cgacgccatc     900

ctgctgagcg acatcctgag agtgaacacc gagatcacca aggcccccct gagcgcctct     960

atgatcaaga gatacgacga gcaccaccag gacctgaccc tgctgaaagc tctcgtgcgg    1020

cagcagctgc ctgagaagta caaagagatt ttcttcgacc agagcaagaa cggctacgcc    1080

ggctacattg acggcggagc cagccaggaa gagttctaca agttcatcaa gcccatcctg    1140

gaaaagatgg acggcaccga ggaactgctc gtgaagctga acagagagga cctgctgcgg    1200

aagcagcgga ccttcgacaa cggcagcatc ccccaccaga tccacctggg agagctgcac    1260

gccattctgc ggcggcagga agatttttac ccattcctga aggacaaccg ggaaaagatc    1320

gagaagatcc tgaccttccg catcccctac tacgtgggcc ctctggccag gggaaacagc    1380

agattcgcct ggatgaccag aaagagcgag gaaaccatca ccccctggaa cttcgaggaa    1440

gtggtggaca agggcgcttc cgcccagagc ttcatcgagc ggatgaccaa cttcgataag    1500

aacctgccca acgagaaggt gctgcccaag cacagcctgc tgtacgagta cttcaccgtg    1560

tataacgagc tgaccaaagt gaaatacgtg accgagggaa tgagaaagcc cgccttcctg    1620

agcggcgagc agaaaaaggc catcgtggac ctgctgttca agaccaaccg gaaagtgacc 1680

gtgaagcagc tgaaagagga ctacttcaag aaaatcgagt gcttcgactc cgtggaaatc 1740

tccggcgtgg aagatcggtt caacgcctcc ctgggcacat accacgatct gctgaaaatt 1800

atcaaggaca aggacttcct ggacaatgag gaaaacgagg acattctgga agatatcgtg 1860

ctgaccctga cactgtttga ggacagagag atgatcgagg aacggctgaa aacctatgcc 1920

cacctgttcg acgacaaagt gatgaagcag ctgaagcggc ggagatacac cggctggggc 1980

aggctgagcc ggaagctgat caacggcatc cgggacaagc agtccggcaa gacaatcctg 2040

gatttcctga agtccgacgg cttcgccaac agaaacttca tgcagctgat ccacgacgac 2100

agcctgacct ttaaagagga catccagaaa gcccaggtgt ccggccaggg cgatagcctg 2160

cacgagcaca ttgccaatct ggccggcagc cccgccatta agaagggcat cctgcagaca 2220

gtgaaggtgg tggacgagct cgtgaaagtg atgggccggc acaagcccga gaacatcgtg 2280

atcgaaatgg ccagagagaa ccagaccacc cagaagggac agaagaacag ccgcgagaga 2340

atgaagcgga tcgaagaggg catcaaagag ctgggcagcc agatcctgaa agaacacccc 2400

gtggaaaaca cccagctgca gaacgagaag ctgtacctgt actacctgca gaatgggcgg 2460

gatatgtacg tggaccagga actggacatc aaccggctgt ccgactacga tgtggaccat 2520

atcgtgcctc agagctttct gaaggacgac tccatcgaca acaaggtgct gaccagaagc 2580

gacaagaacc ggggcaagag cgacaacgtg ccctccgaag aggtcgtgaa gaagatgaag 2640

aactactggc ggcagctgct gaacgccaag ctgattaccc agagaaagtt cgacaatctg 2700

accaaggccg agagaggcgg cctgagcgaa ctggataagg ccggcttcat caagagacag 2760

ctggtggaaa cccggcagat cacaaagcac gtggcacaga tcctggactc ccggatgaac 2820

actaagtacg acgagaatga caagctgatc cgggaagtga aagtgatcac cctgaagtcc 2880

aagctggtgt ccgatttccg gaaggatttc cagttttaca aagtgcgcga gatcaacaac 2940

taccaccacg cccacgacgc ctacctgaac gccgtcgtgg gaaccgccct gatcaaaaag 3000

taccctaagc tggaaagcga gttcgtgtac ggcgactaca aggtgtacga cgtgcggaag 3060

atgatcgcca agagcgagca ggaaatcggc aaggctaccg ccaagtactt cttctacagc 3120

aacatcatga actttttcaa gaccgagatt accctggcca acggcgagat ccggaagcgg 3180

cctctgatcg agacaaacgg cgaaaccggg gagatcgtgt gggataaggg ccgggatttt 3240

gccaccgtgc ggaaagtgct gagcatgccc caagtgaata tcgtgaaaaa gaccgaggtg 3300

cagacaggcg gcttcagcaa agagtctatc ctgcccaaga ggaacagcga taagctgatc 3360

gccagaaaga aggactggga ccctaagaag tacggcggct tcgacagccc caccgtggcc 3420

tattctgtgc tggtggtggc caaagtggaa aagggcaagt ccaagaaact gaagagtgtg 3480

aaagagctgc tggggatcac catcatggaa agaagcagct tcgagaagaa tcccatcgac 3540

tttctggaag ccaagggcta caaagaagtg aaaaaggacc tgatcatcaa gctgcctaag 3600

tactccctgt tcgagctgga aaacggccgg aagagaatgc tggcctctgc cggcgaactg 3660

cagaagggaa acgaactggc cctgccctcc aaatatgtga acttcctgta cctggccagc 3720

cactatgaga agctgaaggg ctcccccgag gataatgagc agaaacagct gtttgtggaa 3780

cagcacaagc actacctgga cgagatcatc gagcagatca gcgagttctc caagagagtg 3840

atcctggccg acgctaatct ggacaaagtg ctgtccgcct acaacaagca ccgggataag 3900

cccatcagag agcaggccga gaatatcatc cacctgttta ccctgaccaa tctgggagcc 3960

cctgccgcct tcaagtactt tgacaccacc atcgaccgga agaggtacac cagcaccaaa 4020

gaggtgctgg acgccaccct gatccaccag agcatcaccg gcctgtacga gacacggatc 4080

gacctgtctc agctgggagg cgactaa 4107

<210> 24
<211> 1399
<212> PRT
<213> artificial

<220>
<223> NLS-His-Cas9 nickase D10A-His protein, modified from
      Streptococcus pyogenes Cas9n

<400> 24

Met Tyr Pro Tyr Asp Val Pro Asp Tyr Ala Ser Pro Lys Lys Lys Arg
1               5                   10                  15

Lys Val Glu Ala Ser Asp Lys Lys Tyr Ser Ile Gly Leu Ala Ile Gly
            20                  25                  30

Thr Asn Ser Val Gly Trp Ala Val Ile Thr Asp Glu Tyr Lys Val Pro
        35                  40                  45

Ser Lys Lys Phe Lys Val Leu Gly Asn Thr Asp Arg His Ser Ile Lys
    50                  55                  60

Lys Asn Leu Ile Gly Ala Leu Leu Phe Asp Ser Gly Glu Thr Ala Glu
65                  70                  75                  80

Ala Thr Arg Leu Lys Arg Thr Ala Arg Arg Arg Tyr Thr Arg Arg Lys
                85                  90                  95

Asn Arg Ile Cys Tyr Leu Gln Glu Ile Phe Ser Asn Glu Met Ala Lys
            100                 105                 110

Val Asp Asp Ser Phe Phe His Arg Leu Glu Glu Ser Phe Leu Val Glu
        115                 120                 125

Glu Asp Lys Lys His Glu Arg His Pro Ile Phe Gly Asn Ile Val Asp
    130                 135                 140

Glu Val Ala Tyr His Glu Lys Tyr Pro Thr Ile Tyr His Leu Arg Lys
145                 150                 155                 160

Lys Leu Val Asp Ser Thr Asp Lys Ala Asp Leu Arg Leu Ile Tyr Leu
                165                 170                 175

Ala Leu Ala His Met Ile Lys Phe Arg Gly His Phe Leu Ile Glu Gly
            180                 185                 190

Asp Leu Asn Pro Asp Asn Ser Asp Val Asp Lys Leu Phe Ile Gln Leu
        195                 200                 205

Val Gln Thr Tyr Asn Gln Leu Phe Glu Glu Asn Pro Ile Asn Ala Ser
    210                 215                 220

Gly Val Asp Ala Lys Ala Ile Leu Ser Ala Arg Leu Ser Lys Ser Arg
225                 230                 235                 240

Arg Leu Glu Asn Leu Ile Ala Gln Leu Pro Gly Glu Lys Lys Asn Gly
                245                 250                 255

Leu Phe Gly Asn Leu Ile Ala Leu Ser Leu Gly Leu Thr Pro Asn Phe
            260                 265                 270

Lys Ser Asn Phe Asp Leu Ala Glu Asp Ala Lys Leu Gln Leu Ser Lys
        275                 280                 285

Asp Thr Tyr Asp Asp Asp Leu Asp Asn Leu Leu Ala Gln Ile Gly Asp
    290                 295                 300

Gln Tyr Ala Asp Leu Phe Leu Ala Ala Lys Asn Leu Ser Asp Ala Ile
305                 310                 315                 320

Leu Leu Ser Asp Ile Leu Arg Val Asn Thr Glu Ile Thr Lys Ala Pro
                325                 330                 335

Leu Ser Ala Ser Met Ile Lys Arg Tyr Asp Glu His His Gln Asp Leu
            340                 345                 350

Thr Leu Leu Lys Ala Leu Val Arg Gln Gln Leu Pro Glu Lys Tyr Lys
        355                 360                 365

Glu Ile Phe Phe Asp Gln Ser Lys Asn Gly Tyr Ala Gly Tyr Ile Asp

370                 375                 380

Gly Gly Ala Ser Gln Glu Glu Phe Tyr Lys Phe Ile Lys Pro Ile Leu
385                 390                 395                 400

Glu Lys Met Asp Gly Thr Glu Glu Leu Leu Val Lys Leu Asn Arg Glu
                405                 410                 415

Asp Leu Leu Arg Lys Gln Arg Thr Phe Asp Asn Gly Ser Ile Pro His
            420                 425                 430

Gln Ile His Leu Gly Glu Leu His Ala Ile Leu Arg Arg Gln Glu Asp
        435                 440                 445

Phe Tyr Pro Phe Leu Lys Asp Asn Arg Glu Lys Ile Glu Lys Ile Leu
    450                 455                 460

Thr Phe Arg Ile Pro Tyr Tyr Val Gly Pro Leu Ala Arg Gly Asn Ser
465                 470                 475                 480

Arg Phe Ala Trp Met Thr Arg Lys Ser Glu Glu Thr Ile Thr Pro Trp
                485                 490                 495

Asn Phe Glu Glu Val Val Asp Lys Gly Ala Ser Ala Gln Ser Phe Ile
            500                 505                 510

Glu Arg Met Thr Asn Phe Asp Lys Asn Leu Pro Asn Glu Lys Val Leu
        515                 520                 525

Pro Lys His Ser Leu Leu Tyr Glu Tyr Phe Thr Val Tyr Asn Glu Leu
    530                 535                 540

Thr Lys Val Lys Tyr Val Thr Glu Gly Met Arg Lys Pro Ala Phe Leu
545                 550                 555                 560

Ser Gly Glu Gln Lys Lys Ala Ile Val Asp Leu Leu Phe Lys Thr Asn
                565                 570                 575

Arg Lys Val Thr Val Lys Gln Leu Lys Glu Asp Tyr Phe Lys Lys Ile
            580                 585                 590

Glu Cys Phe Asp Ser Val Glu Ile Ser Gly Val Glu Asp Arg Phe Asn
        595                 600                 605

Ala Ser Leu Gly Thr Tyr His Asp Leu Leu Lys Ile Ile Lys Asp Lys
    610                 615                 620

Asp Phe Leu Asp Asn Glu Glu Asn Glu Asp Ile Leu Glu Asp Ile Val
625                 630                 635                 640

Leu Thr Leu Thr Leu Phe Glu Asp Arg Glu Met Ile Glu Glu Arg Leu
                645                 650                 655

Lys Thr Tyr Ala His Leu Phe Asp Asp Lys Val Met Lys Gln Leu Lys
            660                 665                 670

Arg Arg Arg Tyr Thr Gly Trp Gly Arg Leu Ser Arg Lys Leu Ile Asn
        675                 680                 685

Gly Ile Arg Asp Lys Gln Ser Gly Lys Thr Ile Leu Asp Phe Leu Lys
    690                 695                 700

Ser Asp Gly Phe Ala Asn Arg Asn Phe Met Gln Leu Ile His Asp Asp
705                 710                 715                 720

Ser Leu Thr Phe Lys Glu Asp Ile Gln Lys Ala Gln Val Ser Gly Gln
                725                 730                 735

Gly Asp Ser Leu His Glu His Ile Ala Asn Leu Ala Gly Ser Pro Ala
            740                 745                 750

Ile Lys Lys Gly Ile Leu Gln Thr Val Lys Val Val Asp Glu Leu Val
        755                 760                 765

Lys Val Met Gly Arg His Lys Pro Glu Asn Ile Val Ile Glu Met Ala
    770                 775                 780

Arg Glu Asn Gln Thr Thr Gln Lys Gly Gln Lys Asn Ser Arg Glu Arg
785                 790                 795                 800

Met Lys Arg Ile Glu Glu Gly Ile Lys Glu Leu Gly Ser Gln Ile Leu
                805                 810                 815

Lys Glu His Pro Val Glu Asn Thr Gln Leu Gln Asn Glu Lys Leu Tyr
            820                 825                 830

Leu Tyr Tyr Leu Gln Asn Gly Arg Asp Met Tyr Val Asp Gln Glu Leu
        835                 840                 845

Asp Ile Asn Arg Leu Ser Asp Tyr Asp Val Asp His Ile Val Pro Gln
    850                 855                 860

Ser Phe Leu Lys Asp Asp Ser Ile Asp Asn Lys Val Leu Thr Arg Ser
865                 870                 875                 880

Asp Lys Asn Arg Gly Lys Ser Asp Asn Val Pro Ser Glu Glu Val Val
                885                 890                 895

Lys Lys Met Lys Asn Tyr Trp Arg Gln Leu Leu Asn Ala Lys Leu Ile
            900                 905                 910

Thr Gln Arg Lys Phe Asp Asn Leu Thr Lys Ala Glu Arg Gly Gly Leu
        915                 920                 925

Ser Glu Leu Asp Lys Ala Gly Phe Ile Lys Arg Gln Leu Val Glu Thr
    930                 935                 940

Arg Gln Ile Thr Lys His Val Ala Gln Ile Leu Asp Ser Arg Met Asn
945                 950                 955                 960

Thr Lys Tyr Asp Glu Asn Asp Lys Leu Ile Arg Glu Val Lys Val Ile
                965                 970                 975

Thr Leu Lys Ser Lys Leu Val Ser Asp Phe Arg Lys Asp Phe Gln Phe
            980                 985                 990

Tyr Lys Val Arg Glu Ile Asn Asn  Tyr His His Ala His  Asp Ala Tyr
        995                 1000                 1005

Leu Asn  Ala Val Val Gly Thr  Ala Leu Ile Lys Lys  Tyr Pro Lys
    1010                 1015                 1020

Leu Glu  Ser Glu Phe Val Tyr  Gly Asp Tyr Lys Val  Tyr Asp Val
    1025                 1030                 1035

Arg Lys  Met Ile Ala Lys Ser  Glu Gln Glu Ile Gly  Lys Ala Thr
    1040                 1045                 1050

Ala Lys  Tyr Phe Phe Tyr Ser  Asn Ile Met Asn Phe  Phe Lys Thr
    1055                 1060                 1065

Glu Ile  Thr Leu Ala Asn Gly  Glu Ile Arg Lys Arg  Pro Leu Ile
    1070                 1075                 1080

Glu Thr  Asn Gly Glu Thr Gly  Glu Ile Val Trp Asp  Lys Gly Arg
    1085                 1090                 1095

Asp Phe  Ala Thr Val Arg Lys  Val Leu Ser Met Pro  Gln Val Asn
    1100                 1105                 1110

Ile Val  Lys Lys Thr Glu Val  Gln Thr Gly Gly Phe  Ser Lys Glu
    1115                 1120                 1125

Ser Ile Leu Pro Lys Arg Asn Ser Asp Lys Leu Ile Ala Arg Lys
    1130                1135                1140

Lys Asp Trp Asp Pro Lys Lys Tyr Gly Gly Phe Asp Ser Pro Thr
    1145                1150                1155

Val Ala Tyr Ser Val Leu Val Val Ala Lys Val Glu Lys Gly Lys
    1160                1165                1170

Ser Lys Lys Leu Lys Ser Val Lys Glu Leu Leu Gly Ile Thr Ile
    1175                1180                1185

Met Glu Arg Ser Ser Phe Glu Lys Asn Pro Ile Asp Phe Leu Glu
    1190                1195                1200

Ala Lys Gly Tyr Lys Glu Val Lys Lys Asp Leu Ile Ile Lys Leu
    1205                1210                1215

Pro Lys Tyr Ser Leu Phe Glu Leu Glu Asn Gly Arg Lys Arg Met
    1220                1225                1230

Leu Ala Ser Ala Gly Glu Leu Gln Lys Gly Asn Glu Leu Ala Leu
    1235                1240                1245

Pro Ser Lys Tyr Val Asn Phe Leu Tyr Leu Ala Ser His Tyr Glu
    1250                1255                1260

Lys Leu Lys Gly Ser Pro Glu Asp Asn Glu Gln Lys Gln Leu Phe
    1265                1270                1275

Val Glu Gln His Lys His Tyr Leu Asp Glu Ile Ile Glu Gln Ile
    1280                1285                1290

Ser Glu Phe Ser Lys Arg Val Ile Leu Ala Asp Ala Asn Leu Asp
    1295                1300                1305

Lys Val Leu Ser Ala Tyr Asn Lys His Arg Asp Lys Pro Ile Arg
    1310                1315                1320

Glu Gln Ala Glu Asn Ile Ile His Leu Phe Thr Leu Thr Asn Leu
    1325                1330                1335

Gly Ala Pro Ala Ala Phe Lys Tyr Phe Asp Thr Thr Ile Asp Arg
    1340                1345                1350

Lys Arg Tyr Thr Ser Thr Lys Glu Val Leu Asp Ala Thr Leu Ile

1355                1360                1365

His Gln Ser Ile Thr Gly Leu Tyr Glu Thr Arg Ile Asp Leu Ser
    1370                1375                1380

Gln Leu Gly Gly Asp Ser Pro Lys Lys Lys Arg Lys Val Glu Ala
    1385                1390                1395

Ser

<210>  25
<211>  4200
<212>  DNA
<213>  artificial

<220>
<223>  NLS-His-Cas9 nickase D10A-His, modified from Streptococcus
       pyogenes Cas9n

<400>  25
atgtacccat acgatgttcc agattacgct tcgccgaaga aaaagcgcaa ggtcgaagcg      60

tccgacaaga agtacagcat cggcctggcc atcggcacca actctgtggg ctgggccgtg     120

atcaccgacg agtacaaggt gcccagcaag aaattcaagg tgctgggcaa caccgaccgg     180

cacagcatca agaagaacct gatcggagcc ctgctgttcg acagcggcga aacagccgag     240

gccacccggc tgaagagaac cgccagaaga agatacacca gacggaagaa ccggatctgc     300

tatctgcaag agatcttcag caacgagatg gccaaggtgg acgacagctt cttccacaga     360

ctggaagagt ccttcctggt ggaagaggat aagaagcacg agcggcaccc catcttcggc     420

aacatcgtgg acgaggtggc ctaccacgag aagtacccca ccatctacca cctgagaaag     480

aaactggtgg acagcaccga caaggccgac ctgcggctga tctatctggc cctggcccac     540

atgatcaagt tccggggcca cttcctgatc gagggcgacc tgaaccccga caacagcgac     600

gtggacaagc tgttcatcca gctggtgcag acctacaacc agctgttcga ggaaaacccc     660

atcaacgcca gcggcgtgga cgccaaggcc atcctgtctg ccagactgag caagagcaga     720

cggctggaaa atctgatcgc ccagctgccc ggcgagaaga agaatggcct gttcggcaac     780

ctgattgccc tgagcctggg cctgaccccc aacttcaaga gcaacttcga cctggccgag     840

gatgccaaac tgcagctgag caaggacacc tacgacgacg acctggacaa cctgctggcc     900

cagatcggcg accagtacgc cgacctgttt ctggccgcca agaacctgtc cgacgccatc     960

ctgctgagcg acatcctgag agtgaacacc gagatcacca aggcccccct gagcgcctct    1020

atgatcaaga gatacgacga gcaccaccag gacctgaccc tgctgaaagc tctcgtgcgg    1080

cagcagctgc ctgagaagta caaagagatt ttcttcgacc agagcaagaa cggctacgcc    1140

ggctacattg acggcggagc cagccaggaa gagttctaca agttcatcaa gcccatcctg 1200

gaaaagatgg acggcaccga ggaactgctc gtgaagctga acagagagga cctgctgcgg 1260

aagcagcgga ccttcgacaa cggcagcatc ccccaccaga tccacctggg agagctgcac 1320

gccattctgc ggcggcagga agatttttac ccattcctga aggacaaccg ggaaaagatc 1380

gagaagatcc tgaccttccg catcccctac tacgtgggcc ctctggccag gggaaacagc 1440

agattcgcct ggatgaccag aaagagcgag gaaaccatca ccccctggaa cttcgaggaa 1500

gtggtggaca agggcgcttc cgcccagagc ttcatcgagc ggatgaccaa cttcgataag 1560

aacctgccca acgagaaggt gctgcccaag cacagcctgc tgtacgagta cttcaccgtg 1620

tataacgagc tgaccaaagt gaaatacgtg accgagggaa tgagaaagcc cgccttcctg 1680

agcggcgagc agaaaaaggc catcgtggac ctgctgttca agaccaaccg gaaagtgacc 1740

gtgaagcagc tgaaagagga ctacttcaag aaaatcgagt gcttcgactc cgtggaaatc 1800

tccggcgtgg aagatcggtt caacgcctcc ctgggcacat accacgatct gctgaaaatt 1860

atcaaggaca aggacttcct ggacaatgag gaaaacgagg acattctgga agatatcgtg 1920

ctgaccctga cactgtttga ggacagagag atgatcgagg aacggctgaa aacctatgcc 1980

cacctgttcg acgacaaagt gatgaagcag ctgaagcggc ggagatacac cggctggggc 2040

aggctgagcc ggaagctgat caacggcatc cgggacaagc agtccggcaa gacaatcctg 2100

gatttcctga agtccgacgg cttcgccaac agaaacttca tgcagctgat ccacgacgac 2160

agcctgacct ttaaagagga catccagaaa gcccaggtgt ccggccaggg cgatagcctg 2220

cacgagcaca ttgccaatct ggccggcagc cccgccatta agaagggcat cctgcagaca 2280

gtgaaggtgg tggacgagct cgtgaaagtg atgggccggc acaagcccga gaacatcgtg 2340

atcgaaatgg ccagagagaa ccagaccacc cagaagggac agaagaacag ccgcgagaga 2400

atgaagcgga tcgaagaggg catcaaagag ctgggcagcc agatcctgaa agaacacccc 2460

gtggaaaaca cccagctgca gaacgagaag ctgtacctgt actacctgca gaatgggcgg 2520

gatatgtacg tggaccagga actggacatc aaccggctgt ccgactacga tgtggaccat 2580

atcgtgcctc agagctttct gaaggacgac tccatcgaca acaaggtgct gaccagaagc 2640

gacaagaacc ggggcaagag cgacaacgtg ccctccgaag aggtcgtgaa gaagatgaag 2700

aactactggc ggcagctgct gaacgccaag ctgattaccc agagaaagtt cgacaatctg 2760

accaaggccg agagaggcgg cctgagcgaa ctggataagg ccggcttcat caagagacag 2820

ctggtggaaa cccggcagat cacaaagcac gtggcacaga tcctggactc ccggatgaac 2880

actaagtacg acgagaatga caagctgatc cgggaagtga aagtgatcac cctgaagtcc 2940

aagctggtgt ccgatttccg gaaggatttc cagttttaca aagtgcgcga gatcaacaac 3000

taccaccacg cccacgacgc ctacctgaac gccgtcgtgg gaaccgccct gatcaaaaag 3060

taccctaagc tggaaagcga gttcgtgtac ggcgactaca aggtgtacga cgtgcggaag 3120

atgatcgcca agagcgagca ggaaatcggc aaggctaccg ccaagtactt cttctacagc 3180

aacatcatga actttttcaa gaccgagatt accctggcca acggcgagat ccggaagcgg 3240

cctctgatcg agacaaacgg cgaaaccggg gagatcgtgt gggataaggg ccgggatttt 3300

gccaccgtgc ggaaagtgct gagcatgccc caagtgaata tcgtgaaaaa gaccgaggtg 3360

cagacaggcg gcttcagcaa agagtctatc ctgcccaaga ggaacagcga taagctgatc 3420

gccagaaaga aggactggga ccctaagaag tacggcggct tcgacagccc caccgtggcc 3480

tattctgtgc tggtggtggc caaagtggaa aagggcaagt ccaagaaact gaagagtgtg 3540

aaagagctgc tggggatcac catcatggaa agaagcagct tcgagaagaa tcccatcgac 3600

tttctggaag ccaagggcta caaagaagtg aaaaaggacc tgatcatcaa gctgcctaag 3660

tactccctgt tcgagctgga aaacggccgg aagagaatgc tggcctctgc cggcgaactg 3720

cagaagggaa acgaactggc cctgccctcc aaatatgtga acttcctgta cctggccagc 3780

cactatgaga agctgaaggg ctcccccgag gataatgagc agaaacagct gtttgtggaa 3840

cagcacaagc actacctgga cgagatcatc gagcagatca gcgagttctc caagagagtg 3900

atcctggccg acgctaatct ggacaaagtg ctgtccgcct acaacaagca ccgggataag 3960

cccatcagag agcaggccga gaatatcatc cacctgttta ccctgaccaa tctgggagcc 4020

cctgccgcct tcaagtactt tgacaccacc atcgaccgga agaggtacac cagcaccaaa 4080

gaggtgctgg acgccaccct gatccaccag agcatcaccg gcctgtacga gacacggatc 4140

gacctgtctc agctgggagg cgacagcccc aagaagaaga gaaaggtgga ggccagctaa 4200

<210> 26
<211> 37
<212> DNA
<213> artificial

<220>
<223> oligonucleotide primer mRFP-F

<400> 26
gcggctagcc accatggcct cctccgagga cgtcatc 37

<210> 27
<211> 37
<212> DNA
<213> artificial

<220>
<223> oligonucleotide primer mRFP-R

<400> 27
gcggctagcg aattcggcgc cggtggagtg gcggccc 37

<210>  28
<211>  31
<212>  DNA
<213>  artificial

<220>
<223>  oligonucleotide primer EGFP-F

<400>  28
gcgggatcca gtgagcaagg gcgaggagct g                                    31


<210>  29
<211>  22
<212>  DNA
<213>  artificial

<220>
<223>  oligonucleotide primer EGFP-R

<400>  29
gtcgcggccg ctttacttgt ac                                              22


<210>  30
<211>  25
<212>  DNA
<213>  artificial

<220>
<223>  oligonucleotide primer gRNA-S1-up

<400>  30
caccgacccc gcctgtaaca cgagc                                           25


<210>  31
<211>  25
<212>  DNA
<213>  artificial

<220>
<223>  oligonucleotide primer gRNA-S1-low

<400>  31
aaactctcgt gttacaggcg gggtc                                           25


<210>  32
<211>  25
<212>  DNA
<213>  artificial

<220>
<223>  oligonucleotide primer gRNA-S2-up

<400>  32
caccgtaccg cagagtctag actcg                                           25


<210>  33
<211>  25

<212>  DNA
<213>  artificial

<220>
<223>  oligonucleotide primer gRNA-S2-low

<400>  33
aaaccgagtc tagactctgc ggtac                                          25


<210>  34
<211>  25
<212>  DNA
<213>  artificial

<220>
<223>  oligonucleotide primer gRNA-X1-up

<400>  34
caccggtctg tgccttctca tctgc                                          25


<210>  35
<211>  25
<212>  DNA
<213>  artificial

<220>
<223>  oligonucleotide primer gRNA-X1-low

<400>  35
aaacgcagat gagaaggcac agacc                                          25


<210>  36
<211>  25
<212>  DNA
<213>  artificial

<220>
<223>  oligonucleotide primer gRNA-X2-up

<400>  36
caccggtaaa gagaggtgcg ccccg                                          25


<210>  37
<211>  25
<212>  DNA
<213>  artificial

<220>
<223>  oligonucleotide primer gRNA-X2-low

<400>  37
aaaccggggc gcacctctct ttacc                                          25


<210>  38
<211>  20
<212>  DNA
<213>  artificial

<220>
<223>  oligonucleotide primer T7-GFP

<400>  38
gaacttcagg gtcagcttgc                                                    20


<210>  39
<211>  20
<212>  DNA
<213>  artificial

<220>
<223>  oligonucleotide primer T7-RFP

<400>  39
aggacggcga gttcatctac                                                    20


<210>  40
<211>  39
<212>  DNA
<213>  artificial

<220>
<223>  deletion clone 1, shown in Fig.12

<400>  40
gaattcacct gctcgtgtcg tggtggactt ctctcagga                               39


<210>  41
<211>  36
<212>  DNA
<213>  artificial

<220>
<223>  deletion clone 2, shown in Figure 12

<400>  41
gaattcacct gctcgtgtta tggacttctc tcagga                                  36


<210>  42
<211>  96
<212>  DNA
<213>  artificial

<220>
<223>  deletion clone 3, shown in Fig.12

<400>  42
gaattcacct gctcgtgtta caggcggggt ttttcttgtt gacaagaatc ctcacaatac        60

cgcagagtct agactcgtgg tggacttctc tcagga                                  96


<210>  43
<211>  26
<212>  DNA
<213>  artificial

<220>

<223>  deletion clone 4, shown in Fig 12

<400>  43
ggactcgtgg tggacttctc tcagga                                            26


<210>  44
<211>  96
<212>  DNA
<213>  artificial

<220>
<223>  target 1 HBV sequence, shown in Fig.12

<400>  44
gaattcacct gctcgtgtta caggcggggt ttttcttgtt gacaagaatc ctcacaatac      60

cgcagagtct agactcgtgg tggacttctc tcagga                                96


<210>  45
<211>  73
<212>  DNA
<213>  artificial

<220>
<223>  Oligonucleotide comprising S1 and S2 gRNA

<400>  45
cctgctcgtg ttacaggcgg ggtttttctt gttgacaaga atcctcacaa taccgcagag      60

tctagactcg tgg                                                         73


<210>  46
<211>  58
<212>  DNA
<213>  artificial

<220>
<223>  Oligonucleotide comprising X1 and X2 gRNA

<400>  46
accacggggc gcacctctct ttacgcggac tccccgtctg tgccttctca tctgccgg        58


<210>  47
<211>  93
<212>  DNA
<213>  artificial

<220>
<223>  primer to clone full length S protein target site, -up

<400>  47
aattcacctg ctcgtgttac aggcggggtt tttcttgttg acaagaatcc tcacaatacc      60

gcagagtcta gactcgtggt ggacttctct cag                                   93


<210>  48
<211>  93
<212>  DNA

<213>  artificial

<220>
<223>  primer to clone full length S protein target site, -low

<400>  48
gatcctgaga gaagtccacc acgagtctag actctgcggt attgtgagga ttcttgtcaa      60

caagaaaaac cccgcctgta acacgagcag gtc                                   93


<210>  49
<211>  64
<212>  DNA
<213>  artificial

<220>
<223>  primer to clone full length X protein target site, -up

<400>  49
aattcaccac ggggcgcacc tctctttacg cggactcccc gtctgtgcct tctcatctgc      60

cggg                                                                   64


<210>  50
<211>  64
<212>  DNA
<213>  artificial

<220>
<223>  primer to clone full length X protein target site, -low

<400>  50
gatccccggc agatgagaag gcacagacgg ggagtccgcg taaagagagg tgcgccccgt      60

ggtg                                                                   64